# EUROPEAN PATENT SPECIFICATION

(11) **EP 0 018 104 B1**
(45) Date of publication and mention of the grant of the patent: **25.05.1983**
(21) Application number: 80300933.1
(22) Date of filing: 25.03.1980
(51) Int. Cl.: C07D 217/26, C07D 221/08, C07D 221/16, C07D 409/06, C07D 491/04, A61K 31/47

(54) **Tetrahydroisoquinolines, their production and the compounds and pharmaceutical compositions containing them for use in the prevention or treatment of hypertension**
Tetrahydroisochinolin-Derivate, ihre Herstellung und sie enthaltende pharmazeutische Zusammensetzungen zur Verwendung bei der Vorbeugung und Behandlung von erhöhtem Blutdruck
Dérivés de la tétrahydro-isoquinoléine, leur préparation, dérivés et compositions pharmaceutiques les contenant pour utilisation dans la prévention et dans le traitement de l'hypertension

(30) Priority: 26.03.1979 JP 35792/79; 08.08.1979 JP 101513/79; 24.10.1979 JP 138020/79
(43) Date of publication of application: 29.10.1980
(73) Proprietor: Takeda Chemical Industries, Ltd., Chuo-ku, Osaka (JP)
(72) Inventor: Oka, Yoshikazu, Kawanishi Hyogo 666 (JP); Nishikawa, Kohei, Nishikyo-ku Kyoto 610-11 (JP)
(74) Representative: Laredo, Jack Joseph

## Description

This invention relates to novel tetrahydroisoquinoline compounds which are useful as pharmaceuticals and a process for producing them.

European Patent Application No. 0012 845, which falls under Art. 54(3) EPC, discloses certain novel tertrahydroisoquinoline compounds of the general formula: 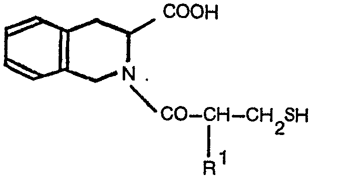 wherein R' is hydrogen or methyl, and pharmaceutically acceptable salts thereof, and processes for preparing them. These compounds are stated to show potent inhibitory activity against the angiotension coverting enzyme (ACE), and to be useful as diagnostic or therapeutic agents for angiotensin-related hypertension.

U.S. Patent No. 4,105,776 relates to certain novel proline derivatives and related compounds which have the general formula: 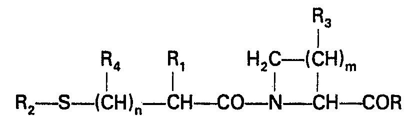 wherein
R is hydroxy, NH₂ or lower alkoxy;
R, and R₄ each is hydrogen, lower alkyl, phenyl or phenyl-lower alkyl;
R₂ is hydrogen, lower alkyl, phenyl, substituted phenyl wherein the phenyl substituent is halo, lower alkyl or lower alkoxy, phenyl-lower alkyl, diphenyl-lower alkyl, triphenyl-lower alkyl, lower alkylthiomethyl, phenyl-lower alkylthiomethyl, lower alkanoyl-amidomethyl, 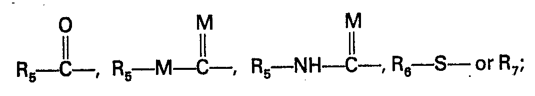
R₃ is hydrogen, hydroxy or lower alkyl;
R₅ is lower alkyl, phenyl or phenyl-lower alkyl;
R₆ is lower alkyl, phenyl, substituted phenyl, (wherein the phenyl substitutent is halo, lower alkyl or lower alkoxy), hydroxy-lower alkyl or amino(carboxy)lower alkyl; 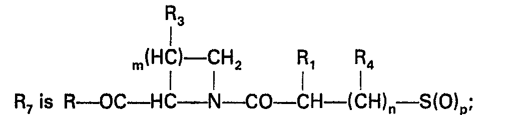
M is O or S;
m is 1 to 3;
n and p each is 0 to 2,

and to processes for making them.

The compounds just defined include both proline and pipecolic acid derivatives. They are also stated to be useful as angiotension converting enzyme inhibitors.

The present invention relates to tetrahydroisoquinoline compounds represented by the formula: 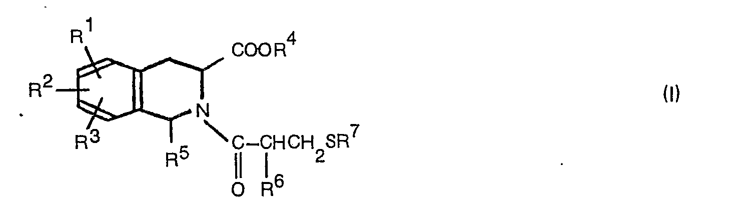 wherein
R¹, R² and R³, which may be the same or different, each represent hydrogen, halogen, nitro, hydroxyl, C₁₋₄ alkyl, C₅₋₇ cycloalkyl, C₁₋₄ alkoxy or 5 to 7 membered cyclic amino selected from pyrrolidinyl, piperidino, homopiperidinyl, piperazinyl and morpholino, with the proviso that, when both R¹ and R² are hydrogen, R³ is one of the substituents defined above except hydrogen, or any two adjacent groups R¹, R² and R³ form C₁₋₄ alkylenedioxy or C₃₋₆ alkylene,
R⁴ and R⁵ each represent hydrogen or C₁₋₄ alkyl,
R⁶ is hydrogen, C₁₋₄ alkyl or ―CH₂SR⁸ in which
R⁸ is hydrogen or C₂₋₄ alkanoyl and
R⁷ is hydrogen, C₂₋₄ alkanoyl or benzoyl, or
R⁷ and R⁸ jointly represent a single bond when
R⁶ is ―CH₂SR⁸,

or a pharmaceutically acceptable salt thereof.

Referring to the above formula (I), the halogen atom represented by R¹, R² and R³ includes, for example, chlorine, bromine, iodine and fluorine, the C₁₋₄ alkyl group represented by R¹, R² and R³ includes, for example, methyl, ethyl, propyl, isopropyl, butyl, isobutyl, or tert-butyl, the C₅₋₇, cycloalkyl group represented by R¹, R² and R³ includes, for example, cyclopentyl, cyclohexyl or cycloheptyl and the C₁₋₄ alkyloxy group represented by R¹, R² and R³ includes, for example, methoxy, ethoxy, propyloxy, isopropyloxy, butyloxy or isobutyloxy. Examples of alkylenedioxy having 1 to 4 carbon atoms include methylenedioxy and ethylenedioxy and of alkylene having 3 to 6 carbon atoms include trimethylene, tetramethylene, pentamethylene and hexamethylene. The C₁₋₄ alkyl group represented by R⁴, R⁵ and R⁶ includes the same alkyl groups as those represented by R¹, R² and R³. The alkanoyl having 2 to 4 carbon atoms represented by R⁷ and R⁸ includes, for example, an acyl group derived from a carboxylic acid such as acetyl, propionyl, butyryl and isobutyryl. Further, when R⁷ and R⁸ jointly represent a single bond, the compound of formula (I) is represented by a compound having a dithiolan ring of the formula: 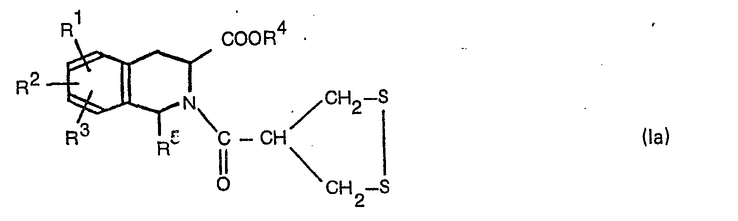 wherein all symbols are as defined above.

Preferred groups as R¹, R² and R³ which may be the same or different, are hydrogen, hydroxyl, C₁₋₄ alkyl or C₁₋₄ alkoxy, or C₁₋₄ alkylenedioxy formed by any two adjacent groups of R¹, R² and R³. More preferably, R¹ is hydrogen and, R² and R³ are hydroxyl or C₁₋₄ alkoxy at the 6- and 7-positions, or R² together with R³ represents methylenedioxy at the 6- and 7-positions; or R¹ and R² are hydrogen and R is C₁₋₄ alkyl at the 7-position.

A preferred group as R⁴ is hydrogen, and a preferred R⁵ is hydrogen or methyl. Regarding R⁶, R⁷ and R⁸, a preferred embodiment is that in which R⁶ is hydrogen, methyl or ―CH₂SR⁸ in which R⁸ is hydrogen or acetyl, and R⁷ is hydrogen or acetyl, or R⁷ together with R⁸ represents a single bond when R⁶ is ―CH₂SR⁸.

The compounds of formula (I) of the present invention can be produced by, for example, reacting a compound of the formula: 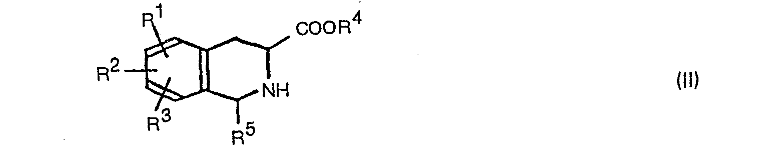 wherein all symbols are as defined above, with a compound represented by the formula: 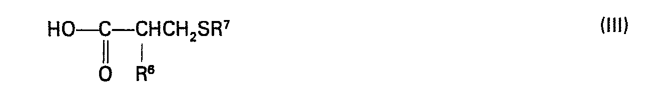 wherein R⁶ and R⁷ are as defined above, or a reactive derivative thereof at the carboxyl functions.

The reactive derivative of the compound (III) at the carboxyl function used in the above reaction between the compounds (II) and (III) includes a derivative, for example, an acid halide such as acid chloride, acid bromide and the like, an acid anhydride obtained by dehydrating one molecule of water from two molecules of the compound (III), a mixed anhydride of the compound (III) wherein the hydrogen atom of carboxyl is substituted with, for example, ethoxycarbonyl, isobutyloxycarbonyl, or benzoyloxycarboxyl.

The reaction can be generally carried out in a suitable solvent and any solvent can be used provided it does not adversely affect the reaction. When the compound (III) is used per se without conversion into its reactive derivative, the reaction is advantageously conducted in the presence of a dehydrating agent, for example, dicyclohexylcarbodiimide. When an acid halide is used as said reactive derivative, the reaction can also be conducted in the presence of a base such as pyridine, picoline, triethylamine, sodium hydroxide, sodium bicarbonate or sodium carbonate. The reaction can be generally carried out at a temperature in the range of from -20°C to +150°C and in most instances, it proceeds sufficiently at ambient temperature.

The compound of the formula (I) wherein R⁷ and/or R⁸ is hydrogen can also be prepared by hydrolysing the compound (I) wherein R⁷ and/or R⁸ is acyl.

The hydrolysis is generally effected in water, an organic solvent or a mixture of these solvents in the absence or presence of an acid, for example, hydrochloric acid, sulfuric acid, nitric acid, phosphoric acid, acetic acid, trifluoroacetic acid, benzenesulfonic acid, methanesulfonic acid, p-toluenesulfonic acid and the like, or a base, for example, ammonia, sodium hydroxide, potassium hydroxide, sodium carbonate: sodium bicarbonate, triethylamine, pyridine and picoline. The hydrolysis is generally conducted at a temperature ranging from -20°C to +150°C, but, in most instances, it proceeds sufficiently at ambient temperature. In this reaction, the carboxylic acid ester at the 3-position of the compound (I) may be simultaneously hydrolysed, but, when the ester form is desired it can be easily, prepared by subjecting the compound obtained after hydrolysis to a conventional esterification procedure.

Contrary to the above, the compound (I) wherein R⁷ and/or R⁸ is acyl can also be prepared by acylating the compound (I) wherein R⁷ and/or R⁸ is hydrogen. The above acylation can be carried out in the same manner as described for the acylation of the compound (II).

Further, the compound of the formula (I) above can also be produced by reacting a compound represented by the formula: 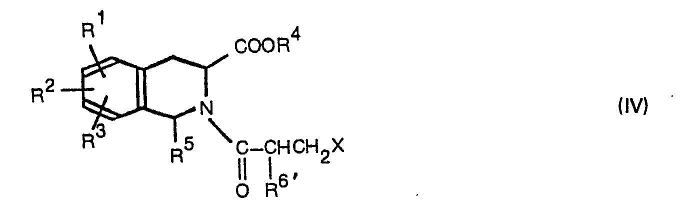 wherein R⁶' represents hydrogen, lower alkyl or -CH₂Y; X and Y each represent halogen or a group represented by R⁹SO₂O― in which R⁹ is C₁₋₄ alkyl, phenyl or p-tolyl; and other symbols are as defined above, with a compound represented by the formula: 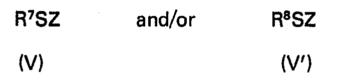 wherein R⁷ and R⁸ are as defined above and Z represents hydrogen or an alkali metal.

In the above process, the lower alkyl group represented by R⁸¹ in the formula (IV) corresponds to a lower alkyl group of R⁶, the halogen represented by X and Y includes chlorine, bromine and iodine, and the alkali metal represented by Z in the formulae (V) and (V') includes lithium, sodium and potassium. The reaction is conducted in a suitable solvent with heating or cooling at a temperature ranging from -20°C to +150°C. When Z is hydrogen, the reaction may proceed advantageously in the presence of a base for example, sodium hydroxide, potassium hydroxide, sodium carbonate, ammonia, pyridine and triethylamine. When R⁷ and R⁸ are different from each other, the compounds (V) and (V') can be reacted simultaneously or in sequence with the compound (IV).

The compound of the formula (I) wherein R⁷ and R⁸ jointly represent a single bond, i.e., the compound represented by the formula (la) can also be prepared by oxidizing the compound of the formula (I) wherein R⁷ and R⁸ are hydrogen.

The above oxidation can be achieved by any one of oxidation procedures using, for example, (I) air or oxygen, (2) halogen such as chlorine, bromine or iodine, (3) peroxides, for example, hydrogen peroxide, sodium peroxide, potassium peroxide, peracetic acid, perbenzoic acid, m-chloroperbenzoic acid, periodic acid, perchloric acid or potassium perchlorate, (4) ferric compounds, for example, potassium ferricyanide or ferric chloride, (5) sodium tetrathionate (Na2s4o,,), (6) sulfoxide compounds, for example, dimethylsulfoxide, or (7) an oxidase, as well as other procedures for oxidizing a mercaptan into a disulfide.

The above oxidation reaction is generally carried out in water, an organic solvent or a mixture thereof, and the organic solvent may be any type of solvent provided it is capable of dissolving the starting materials and it does not adversely affect the reaction. The reaction temperature varies depending upon the oxidation procedure used, but is preferably from -20°C to 100°C and, in most instances, the reaction proceeds adequately at ambient temperature. Also, in order to promote the reaction, a suitable catalyst such as an acid (e.g. hydrochloric acid, sulfuric acid, acetic acid), a base (e.g. ammonia, sodium hydroxide, potassium hydroxide, sodium ethoxide, triethylamine), a metal ion (e.g. a sodium, potassium, calcium, iron, nickel or cobalt ion), silica gel, alumina, activated carbon or diatomaceous earth may be used. In this reaction the compound (I) wherein R⁷ and R⁸ are hydrogen atoms as raw material can be used in the form a salt at the carboxyl moiety and/or the mercapto moiety (for example, a sodium, potassium or magnesium salt).

The desired compound (I) thus obtained can be isolated from the reaction mixture by conventional separation and purification procedures, for example, extraction, concentration, neutralization, filtration, recrystallization, column chromatograpy and thin layer chromatography. When R⁴ represents hydrogen, the compound (I) can be isolated in the form of a free acid or a salt which includes a pharmaceutically acceptable salt such as a salt with an inorganic base (e.g., sodium salt, potassium salt, calcium salt, lithium salt, aluminum salt, magnesium salt, barium salt, ammonium salt, or an organic base addition salt (e.g., methylamine salt, diethylamine salt, triethylamine salt, dicyclohexylamine salt, dibenzylamine salt, hydrazine salt, methylhydrazine salt, phenylhydrazine salt, hydralazine salt, guanidine salt, arginine salt, histidine salt, pyridinium salt, imidazolium salt, quinine salt, cinchonine salt, quinidine salt, cinchonidine salt.

Also, the free acid (I) can be converted into an ester form by a conventional esterification procedure.

The compound represented by the formula (I) according to the present invention has 1 to 3 asymmetric carbons in the molecule and, therefore, may exist as 2 to 8 isomers. Each of these isomers as well as a mixture of these isomers is of course included within the scope of the present invention, and these isomers can be prepared separately if desired. That is, an optical isomer of the compound (I) may be obtained by conducting the above reaction using an optical isomer of either of the starting materials (II) and (III) which have previously been optically resolved. When at least one of (II) and (III) is a racemate, the compound (I).may be generally obtained as a mixture of isomers, but, if desired, these isomers can be separated into each of the isomers in accordance with a usual optical resolution procedure, for example, a method comprising the formation of a salt with an optically active base (e.g., cinchonine, cinchonidine, quinine, or quinidine, of a method comprising chromatography or fractional crystallization.

Each of these optical isomers exhibits the physiological activity described hereinafter, but generally a compound having R-configuration with respect to the carbon at the 1-position, one having S-configuration with respect to the carbon at the 3-position and, when R⁶ is lower alkyl, one having S-configuration with respect to the carbon to which R⁶ is attached, exhibit preferred activities.

The compounds of the present invention, i.e., the tetrahydroisoquinoline compounds represented by the formula (I) and the salts thereof, exhibit inhibitory activities on angiotensin I converting enzyme and bradykinin decomposing enzyme (kininase) in animals, in particular, mammals (for example, human, dog, cat, rabbit, guinea pig and rat), and therefore are useful as agents for dignosis, prevention or treatment of hypertension. Since the compounds of the present invention are of low toxicity, well absorbed on oral administration and highly stable, these compounds can be used safely for the above purpose by oral or parenteral administration, alone or in admixture with suitable pharmaceutically acceptable carriers, excipients or diluents in various pharmaceutical formulations such as powders, granules, tablets, capsules, injectable solutions.

The dosage level generally varies depending upon the conditions to be treated as well as the administration route used, but, for example, in the treatment of hypertension in adult humans, the compounds can be administered orally at a single dose of 0.02 to 20 mg/kg, preferably 0.2 to 2 mg/kg, or intravenously at 0.002 to 0.2 mg/kg, preferably 0.02 to 0.2 mg/kg, 2 to 5 times per day according to the conditions.

The starting compound (II) in the present invention can be easily obtained by, for example, reacting an amino acid or an ester thereof represented by the formula: 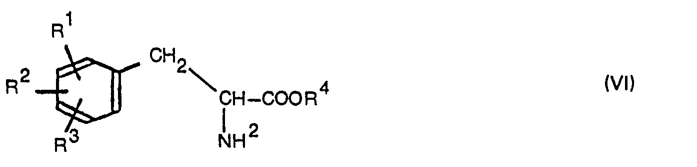 wherein all symbols are as defined above, with an aldehyde represented by the formula: 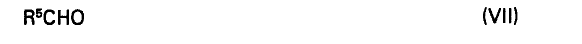 wherein R⁵ is as defined above, according to the method described in the literature (for example, Journal of Organic Chemistry, Vol. 16, p. 430 (1951); ibid, Vol. 26, p. 3533 (1961); Helvetica Chimica Acta, Vol. 55, p. 15 (1972), etc.) or an analogous method.

When any of R', R² and R³ is a cyclic amino group, the corresponding starting compound (II) can also be obtained by, for example, starting from the compound represented by the formula: 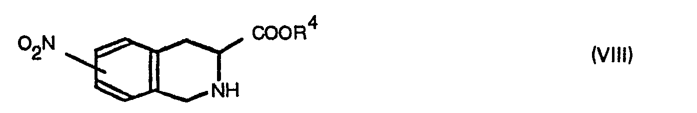 wherein R⁴ is as defined above, through N-acetylation and reduction of the nitro group to produce the compound represented by the formula: 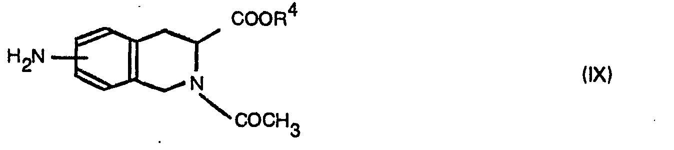 wherein R⁴ is as defined above, converting the amino group into a cyclic amino group in a conventional manner and thereafter hydrolysing the resulting compound.

Further, when any of R', R² and R³ is halogen atom, the corresponding starting compound (II) can also be obtained by diazotizing the amino group in the compound (IX) and halogenating the diazo group in a conventional manner followed by hydrolysis.

The starting compound (III) can be prepared by, for example, an additional reaction of the compound represented by the formula: 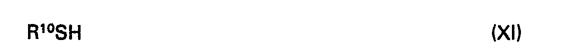 wherein R¹⁰ represents acyl as defined for R⁷, with the compound represented by the formula: 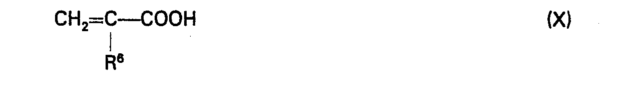 wherein R^{S} is as defined above, and hydrolysis of the resulting product when R⁷ is hydrogen. The addition reaction is carried out in the presence or absence of a suitable solvent, and the solvent can be any type of solvent provided it does not adversely affect the reaction. The reaction temperature may generally be in the range of from -20°C to 150°C, and the reaction proceeds smoothly at room temperature. The hydrolysis may be achieved under the same conditions as those described for the hydrolysis of the compound (I) wherein R⁷ and/or R⁸ is acyl.

When R⁸ is a group represented by -CH₂SR^{B}, the starting compound (III) can be obtained according to the procedure described in the literature (for example, Acta Chemica Scandinavica, Vol. 10, p. 687 (1956)) or an analogous method.

The present invention is further illustrated in greater detail by the following Reference Examples, Examples, Experiments and Preparations.

### Reference Example 1

3.5 g of sodium metal were added to 150 ml of ethanol and, after the sodium was dissolved with stirring, 30 g of diethyl acetamidornalonate and 25 g of p-tert-butylbenzyl chloride were added thereto. Then the mixture was refluxed for 5 hours. 1 I of water was added to the reaction mixture and the solid precipitate was separated by filtration. The solid matter was added to a mixture of 17 g of sodium hydroxide, 100 ml of water and 50 ml of methanol. The whole mixture was heated for 2 hours while the low boiling fraction was removed by distillation. After cooling, the reaction mixture was extracted with 200 ml of diethyl ether, and the aqueous layer was rendered acidic with concentrated hydrochloric acid to precipitate colourless crystals. The crystals collected by filtration were added to a mixture of 150 ml of concentrated hydrochloric acid, 150 ml of water and 200 ml of ethanol, and the whole mixture was refluxed for 7 hours. An additional 100 ml of concentrated hydrochloric acid was added to the mixture, and the whole mixture was heated to distill out 300 ml of the solvent; then, the reaction mixture was cooled to give 27 g of p-tert-butylphenylalanine hydrochloric as colourless scales.
Melting point: 224-227°C (decomposition)
Elemental Analysis for C₁₃H₁₉NO₂·HCl:

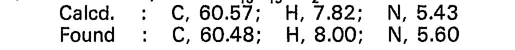

### Reference Example 2

6 g of p-tertbutylphenylalanine hydrochloride produced in Reference Example 1 were dissolved in 100 ml of water by heating at 120°C. To the solution were added 10 ml of formalin. To the mixture solution were then added, 200 ml of concentrated hydrochloric acid dropwise over a period of 30 minutes under reflux. An additional 10 ml of formalin were added to the mixture, and the whole mixture was subjected to heating for 2 hours. The reaction mixture was cooled to give 5 g of 7-tert-butyl-1,2,3,4-tetrahydroisoquinoline-3-carboxylic acid hydrochloride as colourless prisms.
Melting point: 255-260°C (decomposition).
Elemental Analysis for C₁₄H₁₉NO₂·HCl:

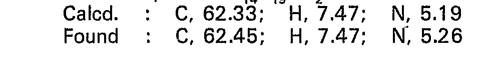

To a mixture of the above product and 50 ml of water was added aqueous ammonia with stirring until the mixture became weakly alkaline. Then, the mixture was rendered weakly acidic with acetic acid to give 4g of free 7-tert-butyl-1,2,3,4-tetrahydroisoquinoline-3-carboxylic acid as colourless crystals.
Melting point: 247-252°C (decomposition)
Elemental Analysis for C₁₄H₁₉NO₂:

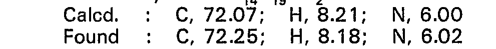

### Reference Example 3

6.9 g of sodium metal were dissolved in 300 ml of ethanol with stirring. To the solution were added 65 g of diethyl acetamidomalonate and 50 g of 6-chloromethyltetraline. The mixture was refluxed for 3 hours. After distilling off the ethanol under reduced pressure to a residual volume of about 1/2, 1 I of water was added to the residue and the precipitate was extracted with ethyl acetate. The extract was washed with water, dried and concentrated under reduced pressure. The residue was crystallized from a mixed solvent of diethyl ether and petroleum ether (1:1 The resulting crude crystals were added to a solution of 17 g of sodium hydroxide in 170 ml of water and the mixture was stirred for 2 hours. After cooling, the mixture was rendered acidic with 10% hydrochloric acid and extracted with ethyl acetate. The extract was washed with water and dried, and the ethyl acetate was distilled off under reduced pressure to give an oily substance. To this oily substance were added 300 ml of 10% hydrochloric acid, and the mixture was refluxed for 7 hours. Then the reaction mixture was concentrated under reduced pressure to a volume of about 1/3. After cooling, the precipitated crystals were collected by filtration to obtain 25 g of 2-amino-3-(5,6,7,8-tetrahydro-2-naphthyl)-propionic acid hydrochloride as colourless needles.
Melting point: 228-230°C
Elemental Analysis for C₁₃H₁₇NO₂·HCl

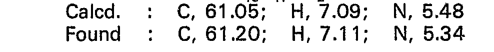

### Reference Example 4

10 g of 2-amino-3-(5,6,7,8-tetrahydro-2-naphthyl)propionic acid hydrochloride obtained in Reference Example 3 and 40 ml of formalin were stirred together at 80°C for 1 hour, followed by addition of 60 ml of concentrated hydrochloric. The mixture was refluxed for 4 hours. The reaction mixture was cooled, then the precipitated crystals were collected by filtration. The crystals were suspended in 50 ml of water. The suspension was rendered weakly alkaline with aqueous ammonia and then rendered weakly acidic with acetic acid to give 7.0 g of free 1,2,3,4,6,7,8,9-octahydrobenzo(g)-isoquinoline-3-carboxylic acid.
inciting point: 291-293°C
Elemental Analysis for C_{'4}H₁₇N0₂

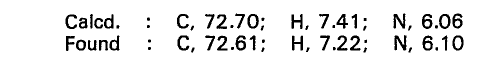

### Reference Example 5

10 g of 2-amino-3-(3,4-methylenedioxyphenyl)propionic acid hydrochloric were stirred with 20 ml of 35% formalin on a water bath at 60-80°C for 1 hour to precipitate 6,7-methylenedioxy-1,2,3,4-tetrahydroisoquinoline-3-carboxylic acid hydrochloride, which was neutralized with aqueous ammonia and the resulting crystals were recrystallized from hot water to give 8 g of crystals of the free amino acid having a melting point of 295°C (decomposition).
Elemental Analvsis for C₁₁H₁₁NO₄:

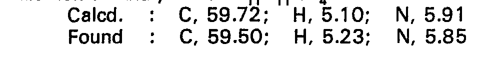

### Reference Examples 6 to 36

The compounds shown in Table 1 were obtained by a procedure of Reference Example 2, 4 or 5, employing, as the respective starting compounds, corresponding α-amino acids prepared by a procedure of Reference Example 1 or 3.

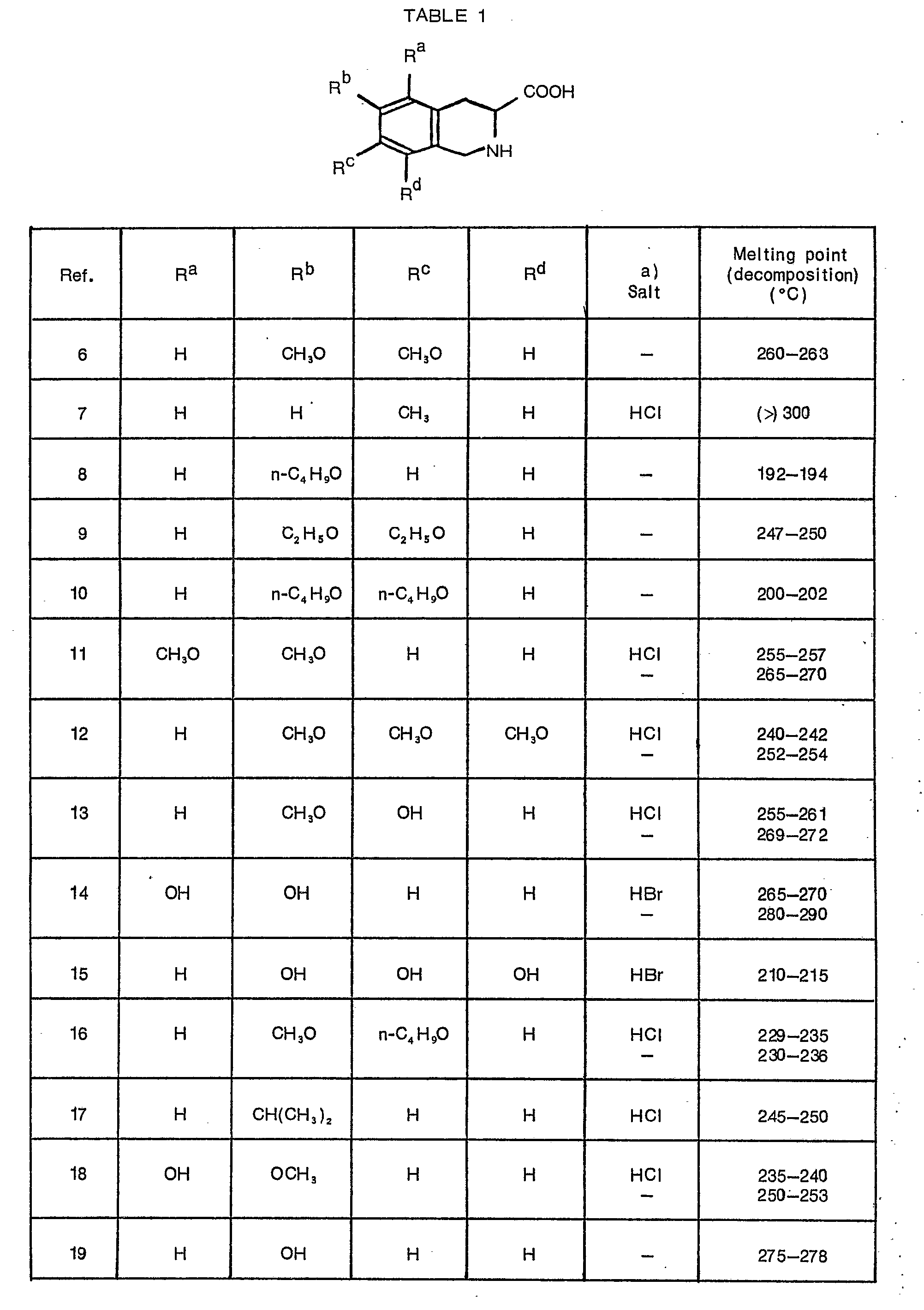

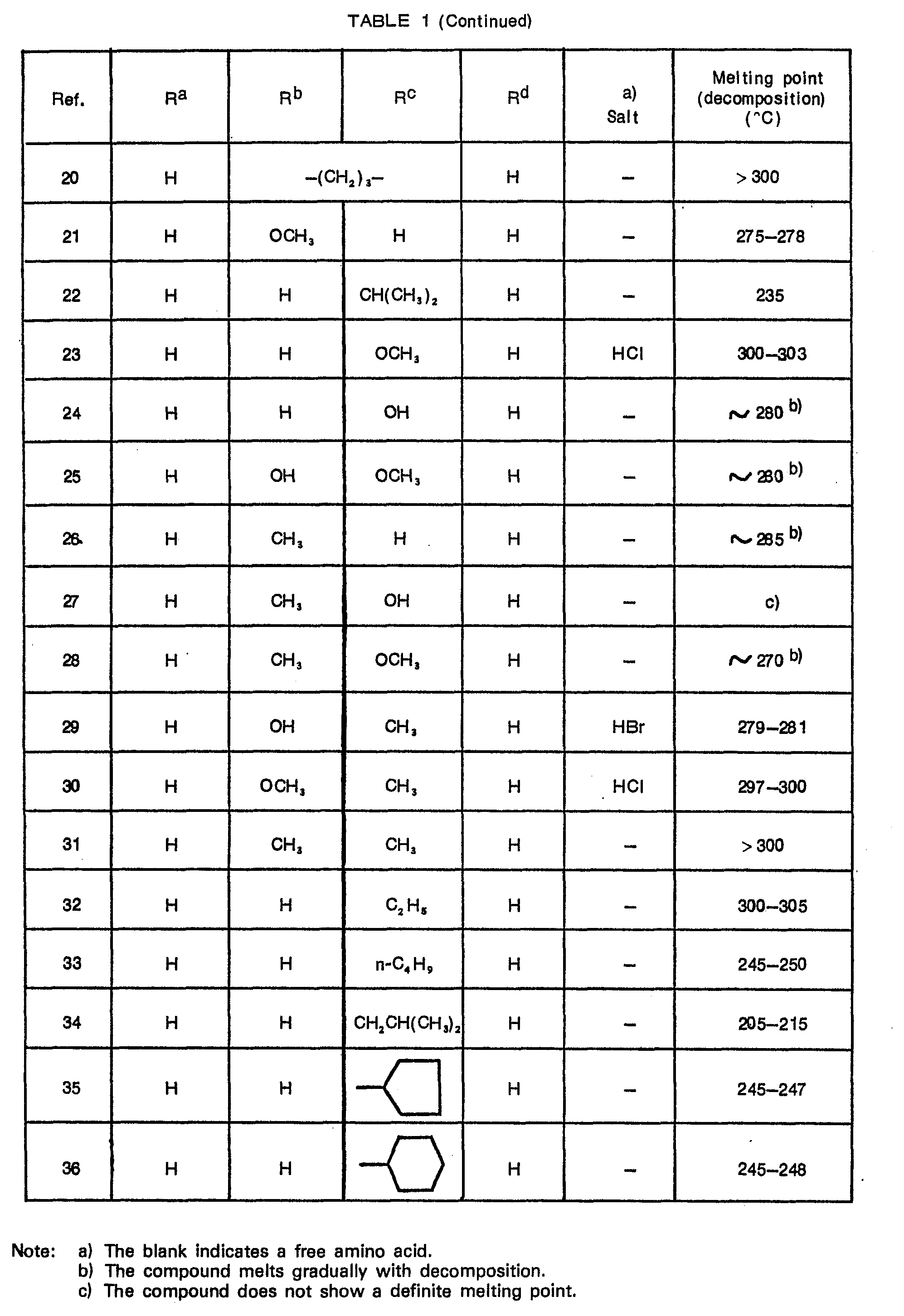

IR: ν^{Nujo}lₘₐₓ 1630 cm⁻¹ (COO⁻)

### Reference Example 37

5 g of 3-(3,4-methylenedioxyphenyl)-L-alanine hydrochloride prepared in accordance with the procedure described in the literature (Chemical & Pharmaceutical Bulletin, Vol. 10, page 680 (1962)) were dissolved in 40 ml of water at 80°C. To the solution were added 10 ml of formalin and 5 ml of concentrated hydrochloric acid, and, after heating at 80°C for 20 minutes was cooled and neutralized with aqueous ammonia. The precipitated crystals were filtered to give 4 g of (S)-6,7-methylene dioxy-1,2,3,4-tetrahydroisoquinoline-3-carboxylic acid.
Melting point: 296-300°C (decomposition).
Optical Rotation: 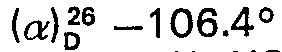 (methanol-ln hydrochloric acid, 2:1 mixed solvent)
Elemental Analysis for C₁₁H₁₁NO₄:

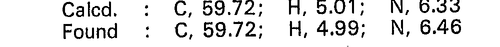

### Reference Examples 38 to 40

The compounds shown in Table 2 were obtained by a reaction similar to that described in Reference Example 37 using an optically active amino acid.

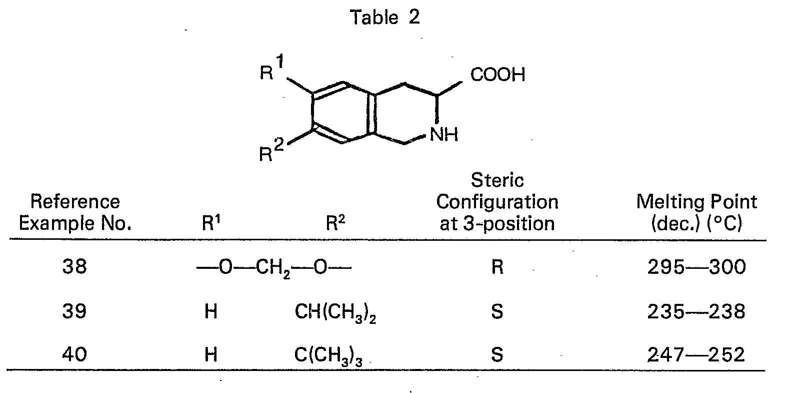

### Reference Example 41

5.0 g of 6,7-methylenedioxy-1,2,3,4-tetrahydroisoquinoline-3-carboxylic acid were suspended in 50 ml of ethanolic hydrochloric acid and, after being refluxed on an oil bath for 7 hours, the solvent was distilled off under reduced pressure. The residue was dissolved in 100 ml of water, made alkaline with aqueous ammonia, and extracted with ethyl acetate. The extract was washed with water, dried and evaporated under reduced pressure to give 4.7 g of ethyl 6,7-methylenedioxy-1,2,3,4-tetrahydroisoquinoline-3-carboxylate as a syrupy substance, which was converted to the hydrochloride, colourless needles with a melting point of 207-209°C.

### Reference Example 42

To 200 ml of concentrated sulfuric acid were added portionwise 100 g of ethyl (S)-1,2,3,4-tetrahydroisoquinoline-3-carboxylate hydrochloride at -20°C, and the mixture was stirred vigorously until a transparent solution was obtained. Thereafter, 36 ml of concentrated nitric acid (d=1.38) were added dropwise thereto while the reaction temperature was kept below 0°C. After the addition was complete, the reaction mixture was stirred for 10 minutes and 2 I of ice water were added thereto. The mixture was rendered alkaline with aqueous ammonia and extracted with dichloromethane. The extract was washed with water, dried over anhydrous sodium sulfate and evaporated. To the residue were added 200 ml of isopropyl ether and the resulting mixture was allowed to stand to give 38 g of crystalline ethyl (S)-7-nitro-1,2,3,4-tetrahydroisoquinoline-3-carboxylate.
Melting point: 97-98°C

### Reference Example 43

5.0 g of ethyl (S)-7-nitro-1,2,3,4-tetrahydroisoquinoline-3-carbonylate was dissolved in 50 ml of 10% hydrochloric acid and the solution was refluxed for 3 hours. The reaction mixture were concentrated under reduced pressure and the residue was dissolved in 20 ml of water. The solution was neutralized with aqueous ammonia to pH 7.0 and the precipitated crystals were filtered, washed with water and dried to give 4.0 g of (S)-7-nitro-1,2,3,4-tetrahydrolsoquinoline-3-carboxylic acid. Melting point: higher than 300°C.

### Reference Example 44

To a solution of 73 g of ethyl (S)-7-nitro-1,2,3,4-tetrahydroisoquinoline-3-carboxylate in 300 ml of dimethylacetamide were added dropwise 27 ml of acetyl chloride under ice-cooling. After stirring at room temperature for 4 hours, the reaction mixture was added to 2 I of ice-water and the mixture was extracted with 500 ml of ethyl acetate. The extract was washed successively with 10% hydrochloric acid and water, dried over anhydrous sodium sulfate and evaporated under reduced pressure. The residue was recrystallized from a mixture of petroleum ether and diethyl ether to give 75 g of ethyl (S)-2-acetyl-7-nitro-1,2,3,4-tetrahydroisoquinoline-3-carboxylate.
Melting point: 70-72°C.

### Reference Example 45

38 g of ethyl (S)-2-acetyl-7-nitro-1,2,3,4-tetrahydroisoquinoline-3-carboxylate were dissolved in 300 ml of ethanol and the solution was catalytically reduced over 4.0 g of 5% palladium-carbon under ambient temperature and atmospheric pressure. After absorption of hydrogen had ceased, the catalyst was removed by filtration and the filtrate was concentrated under reduced pressure. The residue was recrystallized from ethyl acetate to give 20 g of ethyl (S)-2-acetyl-7-amino-1,2,3,4-tetrahydro- isoquinollne-3-carboxylate.
Melting point: 150-152°C.

### Reference Example 46

5 ml of an aqueous solution of 2.9 g of sodium nitrate were added dropwise to 20 ml of a solution of 10 g of ethyl (S)-2-acetyl-7-amino-1,2,3,4-tetrahydroisoquinoline-3-carboxylate in 48% hydrobromic acid under ice-cooling, and the mixture was stirred for 30 minutes. The resulting solution was added dropwise to a suspension of 10 g of cuprous bromide in 60 ml of 48% hydrobromic acid with vigorous stirring at 60°C. After the addition, the mixture was heated at 60°C for an additional hour, and concentrated under reduced pressure. To the residue were added 40 ml of aqueous ammonia and the insoluble substance was filtered off. The filtrate was treated with activated carbon, adusted to pH 7 with acetic acid and concentrated under reduced pressure. The crystals were collected by filtration, washed with water and dried. The obtained crude crystals were dissolved in 20 ml of aqueous ammonia and, after treatment with activated carbon, the solution was adjusted to pH 7.0 and allowed to stand at room temperature. The precipitated crystals were collected by filtration, washed with water and dried to give 5.6 g of (S)-7-bromo-1,2,3,4-tetrahydroisoquinoline-3-carboxylic acid.
Melting point: higher than 300°C

### Reference Example 47

5.0 g of ethyl (S)-2-acetyl-7-amino-1,2,3,4-tetrahydro-3-carboxylate were dissolved in 30 ml of N-methylpyrrolidone and, after addition of 3.0 g of potassium carbonate and 4.2 g of 1,4-dibromobutane, the mixture was stirred at 100°C for 3 hours. The reaction mixture was added to 200 ml of ice-water and extracted with 300 ml of ethyl acetate. The extract was washed with water, dried and concentrated under reduced pressure. The residue was subjected to silica gel column chromatography eluted with acetone-benzene (1:4) to give 5 g of an oily substance. The resulting produce was dissolved in 100 ml of 10% hydrochloric acid, and after being refluxed for 5 hours, the reaction mixture was concentrated under reduced pressure. To the residue were added 100 ml of water, and the mixture was rendered neutral with aqueous ammonia to precipitate crystals, which were then filtered, washed with water and dried to give 3.0 g of (S(-7-pyrrolidinyl-1,2,3,4-tetrahydroisoquinoline-3-carboxylic acid. Melting point: 279-281 °C
Optical Rotation: 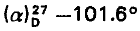 (C=0.58, methanol-IN hydrochloric acid (2:1))

### Reference Example 48

Ethyl (S)-2-acetyl-7-amino-1,2,3,4-tetrahydroisoquinoline-3-carboxylate was reacted with 1,5-dibromopentane in the same manner as described in Reference Example 47 to give (S)-7-piperidino-1,2,3,4-tetrahydroisoquinoline-3-carboxylic acid.
Melting point: 285-268°C
Optical Rotation: 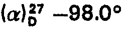 (c=0.77, methanol-1N hydrochloric acid (2:1))

### Reference Example 49

Ethyl (S)-2-acetyl-7-amino-1,2,3,4-tetrahydroisoquinoline-3-carboxylate was reacted with 2,2'-dichloroethyl ether in the same manner as described in Reference Example 47 to give (S)-7-morpholino-1,2,3,4-tetrahydroisoquinoline-3-carboxylic acid.
Melting point: 284-286°C
Optical Rotation: 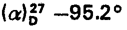 (c=0.89, methanol-1N hydrochloric acid (2:1))

### Example 1

One gram of (S)-6,7-dihydroxy-1,2,3,4-tetrahydroisoquinoline-3-carboxylic acid was suspended in 10 ml of dimethylacetamide, and 1.1 g of 3-acetylthio-2-methylpropionyl chloride was added dropwise thereto over a period of 3 minutes with stirring at room temperature. After the addition was complete, the mixture was stirred at room temperature for 1.5 hours. To the reaction mixture were added 20 ml of water, and the mixture was salted out with sodium chloride. The mixture was then extracted twice with two 20 ml portions each of ethyl acetate. The combined extract was washed with 3 ml of 10% hydrochloric acid and then water, and extracted with a saturated aqueous solution of sodium bicarbonate. The extract was rendered neutral with 10% hydrochloric acid, salted out with sodium chloride and extracted twice with two 20 ml portions each of ethyl acetate. The combined extract was washed with a saturated aqueous solution of sodium chloride, dried over anhydrous sodium ' sulfate, and the solvent was distilled off under reduced pressure. The residue was dissolved in a small amount of chloroform and the solution was poured into a solvent mixture of diethyl ether-petroleum ether (1:1) with stirring to precipitate a colourless amorphous substance. After allowing to stand overnight, the substance was filtered and dried in a desiccator to give 0.7 g of (3S)-2-(3-acetylthio-2-methylpropionyl)-6,7-dihydroxy-1,2,3,4-tetrahydroisoquinoline-3-carboxylic acid as colourless amorphous powder, which showed no definite melting point.

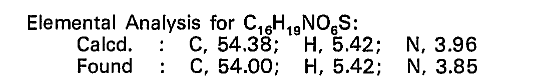

' NMR Spectrum (dimethyl sulfoxide-d₆) δ:
   1.10 (3H, q, CH₃--C), 2.30 (3H, d, CH₃COS), 2.65-3.35 (5H, m), 4.1-4.8 (1 H, m), 5.05 (1H, m), 6.50 (1 H, s, phenyl proton), 6.54 (1 H, s, phenyl proton), 8.67 (2H, s, OHx2), 11.7-13.3 (1 H, br, s, COOH)

In the NMR spectrum, "s" means a singlet, "d" means a doublet, "t" means a triplet, "q" means a quartet, "m" means a multiplet and "br" means a broad absorption (hereinafter the same).
Optical Rotation: 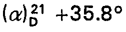 (c=1.13, ethanol)

### Example 2

5 g of 6,7-methylenedioxy-1,2,3,4-tetrahydrolsoquinoline-3-carboxylic acid were suspended in 50 ml of dimethylacetamide and 5.5 g of 3-acetylthio-2-methylpropionyl chloride were added dropwise thereto at room temperature with stirring. After the mixture was stirred for 3 hours at room temperature, 200 ml of water were added thereto and the mixture was extracted with ethyl acetate. The extract was washed successively with 50 ml of 10% hydrochloric acid and water, dried over anhydrous sodium sulfate, and evaporated under reduced pressure. The residue was subjected to column chromatography eluted with a mixture of acetone-benzene (1:4) to give 5.0 g of 2-(3-acetyl- thio-2-methylpropionyl)-6,7-methylenedioxy-1,2,3,4-tetrahydroisoquinoline-3-carboxylic acid as an oily substance, which was obtained as colourless amorphous powder upon drying under reduced pressure, showing no definite melting point.

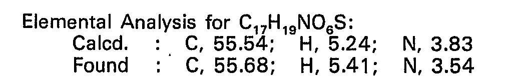

NMR Spectrum (CDCl₃) δ:
   1.10-1.40 (3H, CH₃―C), 2.25―2.30 (3H, m, CH₃COS), 2.90―3.30 (5H, m), 4.65 (2H, m), 5.40 (1 H, m), 5.90 (2H, s, O―CH₂―O), 6.62 (2H, phenyl proton)
Mass Spectrum m/e: 365 (M⁺), 322 (M―COCH₃),

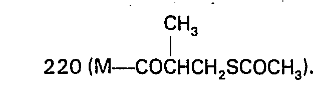

### Example 3

1.5 g of 7-tert-butyl 1,2,3,4-tetrahydroisoquinoline-3-carboxylic acid were suspended in 30 ml of dimethylacetamide and 1.5 g of 3-acetylthio-2-methylpropionyl chloride were added dropwise to the suspension at room temperature with stirring. After allowing to stand overnight at room temperature, the mixture was poured into 200 ml of water, and the product was extracted with 300 ml of ethyl acetate. The organic layer was washed twice with water and dried over anhydrous sodium sulfate, and the solvent was distilled off under reduced pressure. The residue was subjected to silica gel column chromatography and eluted with acetone-benzene (1 :9-1.2) to give 1.5 g of 2-(3-acetylthio-2-methylpropionyl)-7-tert-butyl-1,2,3,4-tetrahydroisoquinoline-3-carboxylic acid, which was obtained a colourless powder upon drying under reduced pressure.

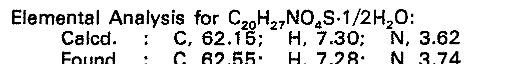

NMR Spectrum (in CDCl₃) δ:
   1.2-1.3 (12H), 2.3―2.5 (3H), 2.8―3.4 (5H), 4.5―5.7 (3H), 7.1―7.4 (3HO, 8.6 (lH)
Mass Spectrum m/e: 377 (M⁺), 334 (M―COCH₃),

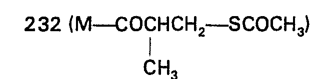

### Example 4

3.0 g of 1, 2,3,4,6,7,8,9-octahydrobenzo(g)isoquinoline-3-carboxylic acid were suspended in 50 ml of dimethylacetamide and 3.3 g of 3-acetylthio-2-methylpropionyl chloride were added dropwise to the suspension at room temperature with stirring. After stirring at room temperature for 3 hours, the mixture was poured into 600 ml of water, and the product was extracted with ethyl acetate. The organic layer was washed successively with 10% HCI and water, and then dried over anhydrous sodium sulfate, and the solvent was distilled off under reduced pressure. The residue was subjected to silica gel column chromatography and eluted with acetone-benzene (1:9). The solvent was distilled off and the residue was dried under reduced pressure to give 3.3 g of 2-(3-acetylthio-2-methylpropionyl)-1,2,3,4,6,7,8,9-octahydrobeno(gj)isoquinoline-3-carboxylic acid as a colourless powder.

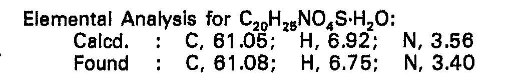

NMR Spectrum (in CDCl₃) δ:
   1.20 (3H, m), 2.80 (4H, s, methylene), 2.30―2.35 (3H), 2.90―3.30 (5H, m), 3.80 (3H, s, OCH₃), 4.10 (sH, q, J=7.5Hz, ―O―CH₂―CH₃), 4.70 (2H, s, CH₂), 5.30-5.45 (1 H, m), 6.80 (1 H, d, J=3Hz, phenyl proton), 7.20 (1 H, d, J=9Hz, phenyl proton), 7.30 (1 H, d, J=9Hz, phenyl proton).
Mass Spectrum m/e: 375 (M⁺), 332 (M-COCH₃),

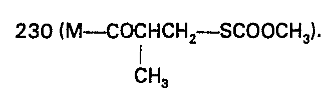

### Example 5

1.1 g of (1R, 3S)-6,7-dihydroxy-methyl-1,2,3,4-tetrahydroisoquinoline-3-carboxylic acid were dissolved in 10 ml of dimethylacetamide and 0.9 g of 3-acetylthio-2-methylpropionyl chloride was added dropwise to the solution at room temperature with stirring. After stirring at room temperature for 5 hours and then allowing to stand overnight, the mixture was poured into 200 ml of water. After addition of 40 g of sodium chloride, the mixture was extracted twice with 100 ml of ethyl acetate. The organic layer was washed once with 1 N hydrochloric acid and twice with water, dried over anhydrous sodium sulfate and evaporated. The residue was subjected to column chromatography using 100 g of silica gel containing 1 g of oxalic acid and eluted with benzene-acetone-methanol (10:10:1). The fractions containing the desired product were collected and evaporated. The residue was dissolved in a small amount of ethyl acetate, and to the solution was added diethyl ether and then petroleum ether to give 0.4 g of (1 R, 3S)-2-(3-acetylthio-2-methylpropionyl)-6,7-dihydroxy-1-methyl-1,2,3,4-tetrahydro- isquinoline-3-carboxylic acid as a colourless powder.

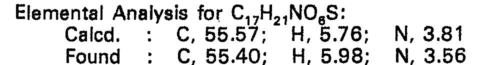

NMR Spectrum (in d₆-DMSO) δ:
   0.8-1.5 (6H, CH₃×2), 2.3 (3H, SCOCH₃), 2.6-3.6 (5H), 4.6-5.2 (2H), 6.5 (2H, phenyl proton), 8.7 (2H, OHx2).
Mass Soectrum m/e: 367 (M⁺). 352 (M―CH₃), 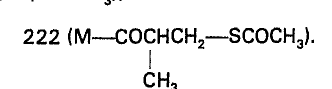
Optical Rotation: 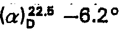 (c=0.4175, methanol)

### Examples 6 to 45

Each of the compounds of the following Examples 6 to 45 was obtained as colourless crystals or colourless powder according to similar procedures to those described in Examples 1 to 5.

### Example 6

2-(3-acetylthio-2-methylpropionyl)-6,7-dimethoxy-1,2,3,4-tetrahydroisoquinoline-3-carboxylic acid.

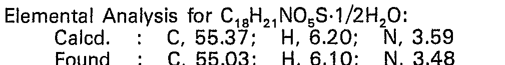

NMR Spectrum (CDCl₃) δ:
   1.08-1.25 (3H, m, CH₃), 2.20-2.26 (3H, m, SCOCH₃), 2.95-3.22 (5H, m), 3.80 (6H, s,
   OCH₃×2), 4.62 (2H, s, CH₂), 5.42 (1H, m), 6.60 (2H, s, phenyl proton), 7.72 (1H, br, COOH).
Mass Spectrum m/e: 381 (M⁺), 338 (M―COCH₃),

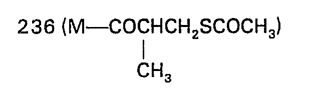

### Example 7

2-(3-acetylthio-2-methylpropionyl)-7-methyl-1,2,3,4-tetrahydroisoquinoline-3-carboxylic acid

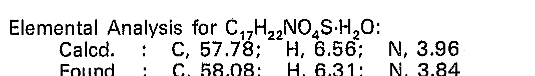

NMR Spectrum (CDCl₃) δ:
   1.15-1.35 (3H, m, CH₃), 2.25-2.35 (6H, m, φ-CH₃ & COCH₃), 2.80-3.30 (5H, m), 4.70 (2H,
   br, s, φ-CH₂―N), 5.40 (1 H, m), 6.85-7.00 (3H, br. s, phenyl proton), 9.90 (1 H, s, COOH).
Mass Spectrum m/e: 335 (M⁺), 292 (M-COCH₃),

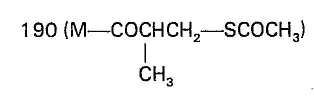

### Example 8

2-(3-acetylthio-2-methylpropionyl)-5,6-dimethoxy-1,2,3,4-tetrahydroisoquinoline-3-carboxylic acid

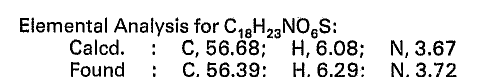

NMR Spectrum (in dimethylsulfoxide-dₛ) 8:
   1.0-1.2 (3H, m, CH₃―C), 2.2-2.3 (3H, m, CH₃COS), 2.7-3.5 (5H, m), 3.7-3.8 (6H, s, CH₃O),
   4.1-5.3 (3H, m), 6.8-7.0 (2H, phenyl proton), 12.5 (1 H, br. COOH).
Mass Spectrum m/e: 381 (M⁺), 338 (M-COCH₃),

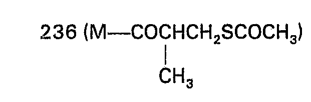

### Example 9

2-(3-acetylthio-2-methylpropionyl)-6-butyloxy-1,2,3,4-tetrahydroisoquinoline-3-carboxylic acid

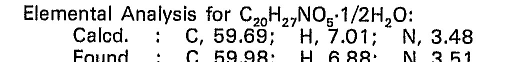

NMR Spectrum (CDCl₃) δ:
   0.95 (3H, t, J=5Hz, CH₃), 1.15-1.26 (3H, m, CH₃), 1.50-1.85 (4H, m, CH₂), 2.30 (3H, m, SCOCH₃), 2.80-3.15 (5H, m, CH₂), 3.90 (2H, t, J=5Hz, O-CH₂), 4.66 (2H, m, CH₂), 5.35 (1 H, m), 6.66 (1 H, s, phenyl proton), 6.73 (1 H, d, J=6Hz, phenyl proton), 7.06 (1 H, d, J=6Hz, phenyl proton).

### Example 10

2-(3-acetylthio-2-methylpropionyl)-6,7,8-trimethoxy-1,2,3,4-tetrahydroisoquinoline-3-carboxylic acid.
Elemental Analysis for C₁₉H₂₅NO₇S:
   Calcd. : C, 55.47; H, 6.13; N, 3.40
   Found : C, 55.20; H, 6.25; N, 3.39
NMR Spectrum (in dimethylsulfoxide-d₆) δ:
   1.0-1.3 (3H, m, CH₃―C), 2.2-2.4 (3H, m, CH₃COS), 2.8-3.3 (5H, m), 3.7-3.9 (9H, s, OCH₃), 4.0-5.3 (3H, m), 6.7 (1H, s, phenyl proton), 12.0 (1H, br. COOH).
Mass Spectrum m/e: 411 (M⁺), 368 (M―COCH₃),

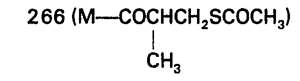

### Example 11

2-(3-acetylthio-2-methylpropionyl)-7-butyloxy-6-methoxy-1,2,3,4-tetrahydroisoquinoline-3-carboxylic acid.
Elemental Analysis for C₂₁H₂₉NO₆S:
   Calcd. : C, 59.56; H, 6.90; N, 3.31
   Found : C, 59.26; H, 6.90; N, 3.13
NMR Spectrum (in dimethylsulfoxide-d₆) δ:
   0.8-1.2 (6H, m, CH₃―C), 1.2-1.8 (4H, m, ―CH₂CH₂―), 2.2―2.4 (3H, m, CH₃COS), 2.7―3.3 (5H, m), 3.7 (3H, s, CH₃O), 3.9 (2H, t, J=6Hz, CH₂0), 4.0-5.3 (3H, m), 6.7-6.8 (2H, s, phenyl protpn), 12.5 (1H, br. COOH).
Mass Spectrum m/e: 423 (M⁺), 380 (M―COCH₃),

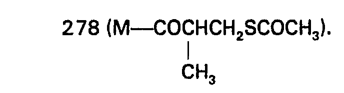

### Example 12

2-(3-acetylthio-2-methylpropionyl)-5,6-dihydroxy-1,2,3,4-tetrahydroisoquinoline-3-carboxylic acid
Elemental Analysis for C₁₆H₁₉NO₆S:
   Calcd. : C, 54.39; H, 5.42; N, 3.96
   Found : C, 54.21; H, 5.73; N, 4.08
NMR Spectrum (in dimethylsulfoxide-dₑ) δ:
   1.0-1.2 (3H, m, CH₃―C), 2.2-2.4 (3H, m, CH₃COS), 2.7-3.5 (5H, m), 4.2-5.2 (3H, m), 6.3―6.7 (2H, m, phenyl proton), 8.3-9.0 (2H, br. OH), 11.5 (1H, br. COOH).
Mass Spectrum m/e: 353 (M⁺), 310 (M―COCH₃),

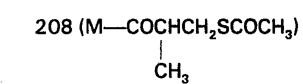

### Example 13

2-(3-acetylthio-2-methylpropionyl)-6,7,8-trihydroxy-1,2,3,4-tetrahydroisoquinoline-3-carboxylic acid.
Elemental Analysis for C₁₆H₁₉NO₇S:
   Calcd. : C, 52.03; H, 5.19; N, 3.79
   Found : C, 51.82; H, 5.47; N, 4.07
_{NMR} Spectrum (in dimethylsulfoxide-d₆) δ:
   1.0-1.2 (3H, m, CH₃―C), 2.2-2.3 (3H, m, CH₃COS), 2.8-3.2 (5H, m), 4.0―5.3 (3H, m), 6.1 (1 H, s, phenyl proton), 8.0-9.0 (3H, br. OH).
Mass Spectrum m/e: 369 (M⁺),

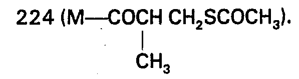

### Example 14

2-(3-acetylthio-2-methylpropionyl)-7-hydroxy-6-methoxy-1,2,3,4-tetrahydroisoquinoline-3-carboxylic acid.
Elemental Analysis for C₁₇H₂₁NO₆S:
   Calcd. : C, 55.58; H, 5.76; N, 3.81
   Found : C, 55.28; H, 5.88; N, 3.75
NMR Spectrum (d₆₋DMSO) 8:
   1.0-1.2 (3H, m, CH₃), 2.2―2.3 (3H, m, SCOCH₃), 2.7―3.3 (5H, m), 3.7 (3H, s, OCH₃), 4.2―5.2 (3H, m), 6.5-6.7 (2H, phenyl proton), 8.7 (1H, br. OH).
Mass Spectrum m/e: 367 (M⁺), 324 (M-COCH₃),

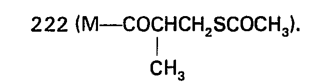

### Example 15

2-(3-acetylthio-2-methylpropionyl)-6,7-diethoxy-1,2,3,4-tetrahydroisoquinoline-3-carboxylic acid.
Elemental Analysis for C₂₀H₂₇NO₆S:
   Calcd. : C, 58.67; H, 6.65; N, 3.42
   Found : C, 58.47; H, 6.57; N, 3.60
NMR Spectrum (CDCl₃) δ:
   1.13-1.25 (3H, m, CH₃), 1.40 (6H, t, J=5Hz, 2×CH₃), 3.00-3.25 (5H, m. CH₂), 4.02 (4H, q J=5Hz, 2×O―CH₂), 4.67 (2H, m, CH₂), 5.45 (1 H, m), 6.50 (2H, s, phenyl proton), 9.18 (1 H, s COOH).

### Example 16

2-(3-acetylthio-2-methylpropionyl)-6,7-dibutyloxy-1,2,3,4-tetrahydroisoquinoline-3-carboxylic acid.
Elemental Analysis for C₂₄H₃₅NO₆S·H₂O:
   Calcd. : C, 59.62; H, 7.71; N, 2.90
   Found : C, 59.91; N, 7.33; N, 2.69
NMR Spectrum (CDCl₃) 8:
   0.98 (6H, t, J=4Hz, 2xCH₃), 1.18-1.30 (3H, m, CH₃), 1.35-1.90 (8H, m, CH₂), 2.20―2.33 (3H, m, SCOCH₃), 2.98-3.30 (5H, m, CH₂), 3.94 (4H, t, J=5Hz, 2xOCH₂), 4.65 (2H, m, CH₂) 5.45 (1 H, m), 6.68 (2H, s, phenyl proton). 9.40 (1 H, s, COOH).

### Example 17

2-(3-acetylthio-2-methylpropionyl)-6-isopropyl-1,2,3,4-tetrahydroisoquinoline-3-carboxylic acid
Elemental Analysis for C₁₉H₂₅NO₄S·H₂O:
   Calcd. : C, 59.83; H, 7.14; N, 3.67
   Found : C, 60.15; H, 7.13; N, 3.43
NMR Spectrum (CDCI₃) δ:
   1.0-1.5 (9H, m, CH₃), 2.2―2.4 (3H, m, SCOCH₃), 2.7-3.3 (6H, m), 4.4―5.4 (3H, m), 6.8―7.1 (3H, m, phenyl proton).
Mass Spectrum m/e: 363 (M⁺), 320 (M―COCH₃),

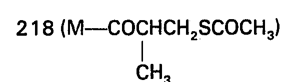

### Example 18

2-(3-acetylthio-2-methylpropionyl)-5-hydroxy-6-methoxy-1,2,3,4-tetrahydroquinoline-3-carboxylic acid
Elemental Analysis for C₁₇H₂₁NO₆S:
   Calcd. : C, 55.58; H, 5.76; N, 3.81
   Found : C, 55.71; H, 5.77; N, 3.86

### Example 19

2-(3-acetylthio-2-methylpropionyl)-6-hydroxy-1,2,3,4-tetrahydroisoquinoline-3-carboxylic acid
Elemental Analysis for C₁₆H₁₉NO₅S·1/2H₂O:
   Calcd. : C, 55.48; H, 5.82; N, 4.04
   Found : C, 55.43; H, 5.87; N, 3.77
Mass Spectrum m/e: 337 (M⁺), 294 (M―COCH₃),

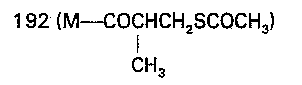

### Example 20

2-(3-acetylthio-2-methylpropionyl)-1,2,3,4,7,8-hexahydro-6H-cyclopenta(g)isoquinoline-3-carboxylic acid
Elemental Analysis for C₁₉H₂₃NO₄S:
   Calcd. : C, 63.14; H, 6.42; N, 3.88
   Found : C, 63.46; H, 6.17; N, 3.57
NMR Spectrum (in CDCl₃) 8:
   1.20 (3H, m), 1.95-2.10 (2H, m), 2.26-2.33 (3H, m), 2.80-3.20 (9H, m), 4.50-4.60 (2H, m), 5.20-5.35 (1H, m), 7.00 (2H, s, phenyl proton), 10.40 (lH, s, COOH).
Mass Spectrum m/e: 361 (M⁺), 318 (M―COCH₃),

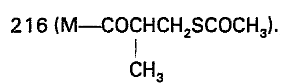

### Example 21

2-(3-acetylthio-2-methylpropionyl)-6-methoxy-1,2,3,4-tetrahydroisoquinoline-3-carboxylic acid
Elemental Analysis for C₁₇H₂₁NO₅S:
   Calcd. : C, 58.11; H, 6.02; N, 3.99
   Found : C, 57.50; H, 6.21; N, 3.74
NMR Spectrum (in CDCI ) δ:
   1.20 (3H, m), 2.25 (3H, d, J=6Hz), 2.90―3.40 (5H, m), 3.33 (3H, s, OCH₃), 4.60―4.80 (2H, m), 5.30-5.43 (1 H, m), 6.66 (1 H, s, phenyl proton), 6.73 (1 H, d, J=9Hz, phenyl proton). 7.03 (1 H, d, J=9Hz, phenyl proton). 9.50 (1 H, s, COOH).
Mass Spectrum m/e: 351 (M⁺), 308 (M-COCH₃),

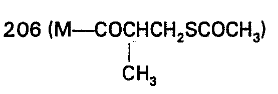

### Example 22

2-(3-acetylthio-2-methylpropionyl)-7-isopropyl-1,2,3,4-tetrahydroisoquinoline-3-carboxylic acid.
Elemental Analysis for C₁₉H₂₅NO₄S:
   Calcd. : C, 62.79; H, 6.93; N, 3.85
   Found : C, 63.04; H, 7.17; N, 3.65
NMR Spectrum (in CDCI ) 8:
   1.20-1.26 (9H, m), 2.20-2.32 (3H, m), 2.70-3.30 (6H, m), 4.70 (2H, m), 5.30-5.46 (1H, m), 6.93-7.10 (3H, m, phenyl proton), 9.60 (1H, s, COOH).
Mass Spectrum m/e: 363 (M⁺), 320 (M―COCH₃),

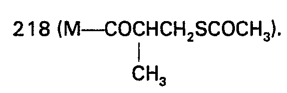

### Example 23

2-(3-acetyhhio-2-methylpropionyll-7-methoxy-1,2,3,4-tetrahydroisoquinoline-3-carboxylic acid.
Elemental Analysis for C₁₇H₂₁NO₅S·1/2H₂O:
   Calcd. : C, 56.66; H, 6.15; N, 3.89
   Found : C, 56.85; H, 6.16; N, 3.65 NMR Spectrum (in CDCl₃) 8:

   1.1-1.3 (3H), 2.2-2.3 (3H), 2.8-3.4 (5H), 3.7 (3H), 4.5-5.7 (3H), 6.7-7.3 (3H), 8.1 (1H)
Mass Spectrum m/e: 351 (M⁺), 308 (M―COCH₃),

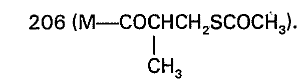

### Example 24

2-(3-acetylthio-2-methylpropionyl)-7-hydroxy-1,2,3,4-tetrahydroisoquinoline-3-carboxylic acid.
Elemental Analysis for C₁₆H₁₉NO₅·1/2H₂O:
   Calcd. : C, 55.49; H, 5.82; N, 4.04
   Found : C, 55.31; H, 6.13; N, 4.46
NMR Spectrum (in CDCl₃ + D₂0) 8:
   1.1-1.3 (3H), 2.2-2.3 (3H, 2.8-3.3 (5H), 4.5-5.5 (3H), 6.4-7.2 (3H).
Mass Spectrum m/e: 337 (M⁺), 294 (M―COCH₃),

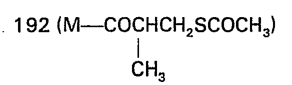

### Example 25

2-(3-acetylthio-2-methylpropionyl)-6-hydroxy-7-methoxy-1,2,3,4-tetrahydroisoquinoline-3-carboxylic acid.
Elemental Analysis for C₁₇H₂₁NO₆S·1/2H₂O:
   Calcd. : C, 54.25; H, 5.89; N, 3.72
   Found : C, 54.25; H, 5.69; N, 3.69
NMR Spectrum (in d₆-DMSO) 8:
   1.0-1.2 (3H), 2.302.4 (3H), 2.7-3.3 (5H), 3.7 (3H), 4.0-5.2 (3H), 6.5-6.8 (2H), 8.8 (1 H).
Mass Spectrum m/e: 367 (M⁺), 324 (M―COCH₃),

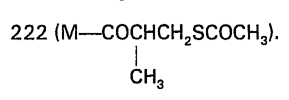

### Example 26

2-(3-acetylthio-2-methylpropionyl)-6-methyl-1,2,2,4-tetrahydroisoquinoline-3-carboxylic acid.
Elemental Analysis for C₁₇H₂₁NO₄S·1/2H₂O:
   Calcd. : C, 59.28; H, 6.44; N, 4.07
   Found : C, 59.28; H, 6.36; N, 3.87
NMR Spectrum (in CDCL₃) 8:
   1.0-1.3 (3H), 2.1-2.5 (6H), 2.7-3.3 (5H), 4.5-5.5 (3H), 6.9―7.1 (3H), 9.2 (1H).
Mass Spectrum m/e: 335 (M⁺), 292 (M-COCH₃),

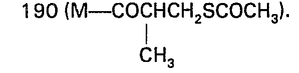

### Example 27

2-(3-acetylthio-2-methylpropionyl)-7-hydroxy-6-methyl-1,2,3,4-tetrahydroisoquinoline-3-carboxylic acid.
Elemental Analysis for C₁₇H₂₁NO₅S:
   Calcd. : C, 58.11; H, 6.02; N, 3.99
   Found : C, 57.53; H, 5.88; N, 3.98
Melting point: Below 200°C with gradual decomposition.
NMR Spectrum (in CDCl₃ + d₆-DMSO + D₂0) 8:
   1.1-1.3 (3H), 2.1 (3H), 2.2-2.4 (3H), 2.7-3.3 (5H), 4.3-5.4 (3H), 6.6 (1H), 6.8 (1H)
Mass Spectrum m/e: 351 (M⁺), 308 (M-COCH₃),

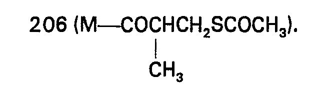

### Example 28

2-(3-acetylthio-2-methylpropionyl)-7-methoxy-6-methyl-1,2,3,4-tetrahydroisoquinoline-3-carboxylic acid.
Elemental Analysis for C₁₈H₂₃NO₅S:
   Calcd. : C, 59.16; H, 6.34; N, 3.83
   Found : C, 59.35; H, 6.48; N, 3.76
Melting point: 119-122°C (Decomposition)

### Example 29

2-(3-acetylthio-2-methylpropionyl)-6-hydroxy-7-methyl-1,2,3,4-tetrahydroisoquinoline-3-carboxylic acid.
Elemental Analysis for C₁₇H₂₁NO₅S:
   Calcd. : C, 58.11; H, 6.02; N, 3.99
   Found : C, 57.85; N, 6.25; N, 3.98
NMR Spectrum (in CDCL + D₂0) 8:
   1.1-1.3 (3H), 2.1 (3H), 2.2-2.4 (3H), 2.7-3.3 (5H), 4.3-5.3 (3H), 6.5-6.9 (2H).
Mass Spectrum m/e: 351 (M⁺), 308 (M--COCH₃),

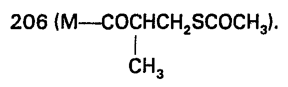

### Example 30

2-(3-acetylthio-2-methylpropionyl)-6-methoxy-7-methyl-1,2,3,4-tetrahydroisoquinoline-3-carboxylic acid.
Elemental Analysis for C₁₈H₂₃NO₅S:
   Calcd. : C, 59.18; H, 6.33; N, 3.83
   Found : C, 59.26; H, 6.44; N, 3.73
Melting point: 144-147°C

### Example 31

2-(3-acetylthio-2-methylpropionyl)-6,7-dimethyl-1,2,3,4-tetrahydroisoquinoline-3-carpoxylic acid.
Elemental Analysis for C₁₈H₂₃NO₄S·1/2H₂O:
   Calcd. : C, 60.32; H, 6.75; N, 3.91
   Found : C, 60.70; H, 6.48; N, 3.96
NMR Spectrum (in CDCl₃) δ:
   1.0-1.3 (3H), 2.1-2.4 (9H), 2.8-3.4 (5H), 4.4-5.5 (3H), 8.7 (1H).
Mass Spectrum m/e: 349 (M⁺), 306 (M―COCH₃),

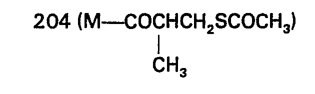

### Example 32

2-(3-acetylthio-2-methylpropionyi)-7-ethyl-1,2,3,4-tetrahydroisoquinoline-3-carboxylic acid.
Elemental Analysis for C₁₈H₂₃NO₄S·1/2H₂O:
   Calcd. : C, 60.32; H, 6.75; N, 3.91
   Found : C, 60.16; H, 6.62; N, 3.56
NMR Spectrum (in CDCI₃ + D₂0) δ:
   1.0-1.4 (6H), 2.2-2.4 (3H), 2.4-3.4 (7H), 4.4-5.5 (3H), 6.8-7.2 (3H).
Mass Spectrum m/e: 349 (M⁺), 306 (M―COCH₃),

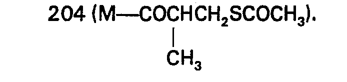

### Example 33

2-(3-acetylthio-2-methylpropionyl)-7-n-butyl-1,2,3,4-tetrahydroisoquinoline-3-carboxylic acid.
Elemental Analysis for C₂₀H₂₇NO₄S:
   Calcd. : C, 63.64; H, 7.21; N, 3.71
   Found : C, 63.34; H, 7.27; N, 3.74
NMR Spectrum (in CDCl₃ + D₂0) 8:
   0.8-1.7 (11H), 2.2-2.4 (3H), 2.4-2.4 (7H), 4.3-5.4 (3H, 6.8-7.2 (3H).
Mass Spectrum m/e: 377 (M⁺), 334 (M―COCH₃),

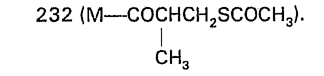

### Example 34

2-(3-acetylthio-2-methylpropionyl)-7-isobutyl-1,2,3,4-tetrahydroisoquinoline-3-carboxylic acid.
Elemental Analysis for C₂₀H₂₇NO₄S:
   Calcd. : C, 63.64; H, 7.21; N, 3.71
   Found : C, 63.34; H, 7.44; N, 3.45
NMR Spectrum (in CDCl₃ + D₂0) 8:
   0.8 (3H), 0.9 (3H), 1.1-1.3 (3H), 1.6-2.0 (1 H), 2.2-2.5 (5H), 2.8-3.3 (5H), 4.4-5.4 (3H), 6.8
Mass Spectrum m/e: 377 (M⁺), 334 (M-COCH₃),

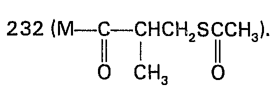

### Example 35

2-(3-acetylthio-2-methylpropionyl)-7-cyclopentyl-1,2,3,4-tetrahydroisoquinoline-3-carboxylic acid.
Elemental Analysis for C₂₁H₂₇NO₄S·1/2H₂O:
   Calcd. : C, 63.30; H, 7.04; N, 3.52
   Found : C, 63.37; H, 7.05; N, 3.38
NMR Spectrum (in CDCl₃) 8:
   1.20-1.30 (3H, m), 1.60-2.00 (8H, m), 2.23-2.33 (3H, m SCOCH₃), 2.80-3.30 (6H, m), 4.60-4.90 (2H, m), 5.30-5.50 (1H, m), 7.00-7.10 (3H, m, phenyl proton), 8.40 (lH, s, COOH).
Mass Spectrum m/e: 389 (M⁺), 346 (M―COCH₃),

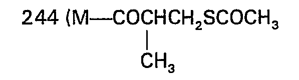

### Example 36

2-(3-acetylthio-2-methylpropionyl)-7-cyclohexyl-1,2,3,4-tetrahydroisoquinoline-3-carboxylic acid.
Elemental Analysis for C₂₂H₂₉NO₄S·1/2H₂O:
   Calcd. : C, 64.05; H, 7.33; N, 3.40
   Found : C, 64.01; H, 7.34; N, 3.28
NMR Spectrum (in CDCl₃) 8:
   1.20-1.33 (9H, m), 1.80 (1 H, s, methylene), 2.20-2.33 (3H, m, SCOCH₃), 2.90-3.33 (6H, m), 4.60-4.70 (2H, m), 5.30-5.46 (1 H, m), 6.96-7.03 (3H, m, phenyl proton), 9.63 (1 H, s, COOH).
Mass Spectrum m/e: 403 (M), 360 (M―COCH₃),

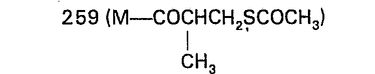

### Example 37

(3H)-2-(3-acetylthio-2-methylpropionyl)-7-tert-butyl-1,2,3,4-tetrahydroisoquinoline-3-carboxylic acid.
Elemental Analysis for C₂₀H₂₇NO₄S·1/2H₂O:
   Calcd. : C, 62.15; H, 7.30; N, 3.62
   Found : C, 62,36; H, 7.77; N, 4.08
Optical Rotation: 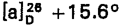 (c=1.1, methanol)

### Example 38

(3S)-2-(3-acetylthio-2-methylpropionyl)-6,7-methylenedioxy-1,2,3,4-tetrahydroisoquinoline-3-carboxylic acid.
Elemental Analysis for C₁₇H₁₉NO₆S:
   Calcd. : C, 55.89; H, 5.24; N, 3.83
   Found : C, 55.70; H, 5.25; N, 3.66
Optical Rotation: 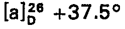 (c=1.2, methanol)

### Example 39

(3R)-2-(3-acetylthio-2-methylpropionyl)-6,7-methylenedioxy-1,2,3,4-tetrahydroisoquinoline-3-carboxylic acid.
Elemental Analysis for C₁₇H₁₉NO₆S:
   Calcd. : C, 55.89; H, 5.24; N, 3.83
   Found C, 55.59; H, 5.29; N, 4.07
Optical Rotation: 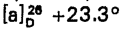 (c=0.52, methanol)

### Example 40

(3S)-2-13-acetylthio-2-methylpropionyl)-7-isopropyl-1,2,3,4-tetrahydroisoquinoline-3-carboxylic acid.
Elemental Analysis for C₁₉H₂₅NO₄S:
   Calcd. : C, 62.79; H, 6.93; N, 3.85
   Found : C, 62.38; H, 6.96; N, 3.84
Optical Rotation: 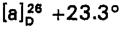 (c=0.52, methanol)

### Example 41

2-(3-acetylthio-2-methylpropionyl)-7-nitro-1,2,3,4-tetrahydroisoquinoline-3-carboxylic acid.
Elemental Analysis for C₁₆H₁₈N₂O₆S:
   Calcd. : C, 52.46; H, 4.95; N, 7.65
   Found : C, 52.30; H, 4.68; N, 7.57
NMR Spectrum (in CDCl₃) δ:
   1.15-1.30 (3H, m, CH₃), 2.30-2.40 (3H, m, SCOCH₃), 2.80-3.50 (5H, m), 4.80―4.95 (2H, m), 5.40-5.55 (1 H, m), 7.33 (1 H, d, J=9Hz, phenyl proton), 7.90₋8.15 (2H, m, phenyl proton), 9.40 (1 H, s, COOH).
Mass Spectrum m/e: 366 (M⁺), 323 (M―COCH₃),

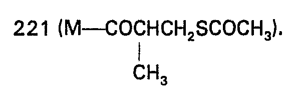

### Example 42

2-(3-acetylthio-Z-methylpropionyl)-7-bromo-1,2,3,4-tetrahydroisoquinoline-3-carboxylic acid.
Elemental Analysis for C₁₆H₁₈NO₄Br:
   Calcd. : C, 48.01; H, 4.53; N, 3.50
   Found : C, 48,17; H, 4.76; N, 3.76
NMR Spectrum (in CDCI₃) δ:
   1.20―1.30 (3H, m, CH₃), 2.26-2.33 (3H, m, SCOCH₃), 2.90-3.30 (5H, m), 4.66-480 (2H, m), 5.30-5.50 (1H, m), 7.00―7.33 (3H, m, phenyl proton), 9.10 (1H, s, COOH).
Mass Spectrum m/e: 399 (M⁺), 356 (M―COCH₃),

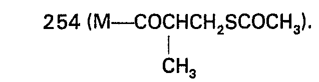

### Example 43

2-(3-acetylthio-2-methylpropionyl)-7-(1-pyrrolidinyl)-1,2,3,4-tetrahydroisoquinoline-3-carboxylic acid.
Elemental Analysis for C₂ₒH₂₆N₂O₄S:
   Calcd. : C, 61.52; H, 6.71; N, 7.18
   Found : C, 61.75 H, 6.89; N, 6.84
NMR Spectrum (in CDC1₃) 8:
   1.10―11.30 (3H, m), 1.90-2.03 (4H, m), 2.20-2.26 (3H, m, SCOCH₃), 2.80-3.30 (9H, m), 4.66 (2H, s), 5.20-5.40 (1 H, m), 6.32 (1 H, s, phenyl proton), 6.43 (1 H, d, J=9Hz, phenyl proton), 6.96 (1 H, d, J=9Hz, phenyl proton), 9.10 (1H, s, COOH).
Mass Spectrum m/e: 390 (M⁺), 347 (M―COCH₃),

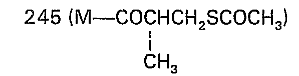

### Example 44

2-(3-acetylthio-2-methylpropionyl)-7-piperidino-1,2,3,4-tetrahydroisoquinoline-3-carboxylic acid.
Elemental Analysis for C₂₁H₂₈N₂O₄S:
   Calcd. : C, 62.36; H, 6.98; N, 6.93
   Found : C, 62.06; H, 6.75; N, 6.77
NMR Spectrum (in CDCl₃) δ:
   1.15-1.30 (3H, m), 1.60 (6H, m), 2.20-2.31 (3H, m), 3.00-3.20 (9H, m), 4.65-4.75 (2H, m), 5.30-5.46 (1 H, m), 6.73 (1 H, s, phenyl proton), 6.80 (1 H, d, J=9Hz, phenyl proton), 7.03 (1 H, d, J=Hz, phenyl proton), 8.90 (1 H, s, COOH).

### Example 45

2-(3-acetylthio-2-methylpropionyl)-7-morpholino-1,2,3,4-tetrahydroisoquinoline-3-carboxylic acid hydrochloride.
Elemental Analysis for C₂₀H₂₆N₂O₅S·HCl·H₂O:
   Calcd. : C, 53.15; H, 6.25; N, 6.20
   Found : C, 53.31; H, 6.09; N, 6.27
NMR Spectrum (in d₆-DMSO) 8:
   1.00-1.13 (3H, rn), 2.23―2.33 (3H, m), 2.90―3.10 (5H, m), 3.23―3.40 (4H, m), 3.95 (4H, s), 4.60-4.80 (2H, m), 5.05-5.25 (1H, m), 7.30-7.40 (3H, m, phenyl proton) 8.10 (1H, s, COOH).
Mass Spectrum m/e: 406 (M⁺), 363 (M―COCH₃),

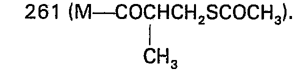

### Example 46

2.1 g of (S)-6,7-dihydroxy-1,2,3,4-tetrahydroisoquinoline-3-carboxylic acid were suspended in 10 ml of dimethylacetamide and 1.7 g of 3-acetylthiopropionyl chloride were added dropwise to the suspension with ice-cooling and stirring. After stirring at room temperature for 7 hours, the reaction mixture was poured into 200 ml of water and 40 g of sodium chloride were added thereto. The mixture was extracted three times with ethyl acetate (100 ml, 50 ml and' 50 ml, respectively). The combined extract was washed successively with 100 ml of 1 N hydrochloric acid and 50 ml of saturated aqueous solution of sodium chloride and extracted with 50 ml of 4% aqueous sodium bicarbonate solution. The extract was neutralized with hydrochloric acid, and the precipitate was extracted with ethyl acetate. The extract was washed successively with water and a saturated aqueous solution of sodium chloride and, after drying over anhydrous sodium sulfate, the solvent was distilled off. The residue was then purified by subjecting it to column chromatography packed with 100 g of silica gel to which 1 g of oxalic acid had been absorbed, and eluting with a mixed solvent of benzene-acetone-methanol (4:4:1) to give 0.4 g of (S)-2-(3-acetyl-thiopropionyl)-6,7dihydroxy-1,2,3,4-tetrahydroisoquinoline-3-carboxylic acid as colourless amorphous powder.
Elemental Analysis for C₁₅H₁₇NO₆S·1/2H₂O:
   Calcd. : C, 51.71; H, 5.21; N, 4.02
   Found : C, 51.74; H, 5.30; N, 3.93
Mass Spectrum m/e: 339 (M⁺), 296 (M―COCH₃), 208 (M―COCH₂CH₂SCOCH₃).
NMR Spectrum (dimethyl sulfoxide-d₆) 8:
   2.33 (3H, s, SCOCH₃), 2.6-3.2 (6H, m), 4.00-5.20 (3H, m), 6.50 (2H, s, phenyl proton), 8.70 (2H, s, OHx2), 12.5 (1H, br. s, COOH)
Optical Rotation: 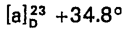 (c=0.825, methanol.

### Examples 47 and 48

The compounds of Examples 47 and 48 were obtained in the same manner as described in Example 46.

### Example 47

(IB,3S)-2-(3-acetylthiopropionyl)-6,7-dihydroxy-1-methyl-1,2,3,4-tetrahydroisoquinoline-3-carboxylic acid.
Elemental Analysis for C₁₆H₁₉NO₆S·1/2H₂O:
   Calcd. : C, 53.03; H, 5.56; N, 3.87
   Found : C, 52.87; H, 5.70; N, 3.60 (S)
NMR Spectrum (in dₑ₋DMXO) 8:
   1.2 (3H, C₁―CH₃), 2.3 (3H, SCOCH₃), 2.5-3.6 (6H, CH₂x3), 4.6-5.2 (2H, C,-H, C₃―H), 6.5 (2H, phenyl proton), 8.6 (2H, OHx2).
Mass Spectrum m/e: 353 (M⁺), 338 (M-CH₃), 222 (M―COCH₂CH₂SCOCH₃).
Optical Rotation: 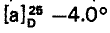 (c=0.425, methanol)

### Example 48

2-(3-acetylthiopropionyl)-7-tert-butyl-1,2,3,4-tetrahydroisoquinoline-3-carboxylic acid.
Elemental Analysis for C₁₉H₂₅NO₄S:
   Calcd. : C, 62.79; H, 6.93; N, 3.85
   Found : C, 62.38; H, 6.92; N, 3.85
NMR Spectrum (n CDCl₃), δ:
   1.3 (9H), 2.3 (3H), 2.6-3.3 (6H), 4.3-5.4 (3H), 7.0-7.3 (3H).
Mass Spectrum m/e: 363 (M⁺), 320 (M-COCH₃), 232 (M―COCH₂CH₂SCOCH₃).

### Example 49

3.0 g of ethyl 6.7-methylenedioxy-1,2,3,4-tetrahydroisoquinoline-3-carboxylate were dissolved In 30 ml of dimethylacetamide and 3.3 g of 3-acetylthio-2-methylpropionyl chloride were added dropwise to the solution, and the mixture was stirred for 2 hours at room temperature. After addition of 10 ml of water, the mixture was extracted with ethyl acetate. The extract was washed with 10 ml of 10% hydrochloric acid, washed with water and dried. The solvent was distilled off, and the residue was subjected to silica gel column chromatography eluted with a mixed solvent of acetone-benzene (1:20) to give 2.7 g of ethyl 2-(3-acetylthio-2-methylpropionyl)-6.7-methylenedioxy-1,2,3,4-tetrahydroisoquinoline-3-carboxylate as an oily substance.
Elemental Analysis for C₁₉H₂₃NO₆S:
   Calcd. : C, 58.01; H, 5.89; N, 3.56
   Found : C, 58.13; H, 5.71; N, 3.40
NMR Spectrum (CDCl₃) δ:
   1.05―1.35 (6H, m, CH₃x2), 2.30-2.34 (3H, m, SCOCH₃), 2.95-3.26 (6H, m), 4.10 (2H, q, J=7Hz, OCH₂CH₃), 4.66 (2H, s, CH₂), 5.35 (1 H, m), 5.90 (2H, s, CH₂), 6.60 (2H, s, phenyl proton).

### Example 50

₁ g of 2-(3-acetylthio-2-methylpropionyl)-7-tert-butyl-1,2,3,4-tetrahydroisoquinoline-3-carboxylic acid obtained as described in Example 3 was dissolved in 20 ml of methanol and 30 ml of 5.5N methanolic ammonia were added to the solution at room temperature. After allowing to stand for 1.5 hours, the reaction mixture was distilled under reduced pressure and 50 ml of water were added to the residue. After rendering the mixture acidic with 10% hydrochloric acid, the mixture was extracted with 200 ml of ethyl acetate. The extract was dried over anhydrous sodium sulfate and distilled under reduced pressure. The residue was subjected to silica gel column chromatography and eluted with acetone-benzene (1:4-1:1) to give 7-tert-butyl-2-(3-mercapto-2-methylpropionyl)-1,2,3,4-tetrahydroisoquinoline-3-carboxylic acid. The product was crystallized from a mixed solvent of diethyl ether-petroleum ether to afford colourless prisms. Yield, 0.35 g.
Melting point, 177-183°C (decomposition).
Elemental Analysis for C₁₈H₂₅NO₃S:
   Calcd. : C, 64.46; H, 7.51; N, 4.18
   Found : C, 64.48; H, 7.53; N, 4.10
NMR Spectrum (in CDCI + D₂O) 8:
   1.1-1.3 (12H), 2.3―3.3 (5H), 4.5-5.5 (3H), 7.0-7.3 (3H).
Mass Spectrum m/e: 335 (M⁺),

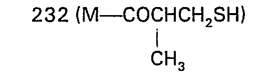

### Examples 51 to 54

Each of the compounds of Examples 51 to 54 was obtained as colourless powder by the same reaction procedure as described in Example 50.

### Example 51

(3S)-7-tert-butyl-2-(3-mercapto-2-methylpropionyl)-1,2,3,4-tetrahydroisoquinoline-3-carboxylic acid.
Elemental Analysis for C₁₈H₂₅NO₃S:
   Calcd. : C, 64.46; H, 7.51; N, 4.18
   Found : C, 64.09; H, 7.52; N, 4.00
Optical Rotation: 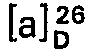 +15.7° (c=0.26, in methanol)

### Example 52

(3S)-2-(3-mercapto-2-methylpropionyl)-6,7-methylenedioxy-1,2,3,4-tetrahydroisoquinoline-3-carboxylic acid.
Elemental Analysis for C₁₅H₁₇NO₅S:
   Calcd. : C, 55.72; H, 5.30; N, 4.33
   Found : C, 56.00; H, 5.39; N, 4.19
Optical Rotation: 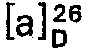 +36.1 (c=0.67, in methanol)

### Example 53

(3S)-7-isopropyl-2-(3-mercapto-2-methylpropionyl)-1,2,3,4-tetrahydroisoquinoline-3-carboxylic acid.
Elemental Analysis for C₁₇H₂₃NO₃S:
   Calcd. : C, 63.53; H, 7.21; N, 4.36
   Found : C, 63.63; H, 7.09; N, 4.04
Optical Rotation: [a]²⁶ +23.7° (c=0.60, in methanol)

### Example 54

2-(3-mercapto-2-methylpropionyl-6,7-methylenedioxy-1,2,3,4-tetrahydroisoquinoline-3-carboxylic acid.
Elemental Analysis for C₁₅H₁₇NO₅S:
   Calcd. : C, 55.72; H, 5.30; N, 4.33
   Found : C, 55.89; H, 5.28; N, 4.13
Mass Spectrum m/e: 323 (M⁺), 278 (M--COOH),

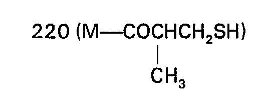

### Example 55

2.1 g of (S)-6,7-dihydroxy-1,2,3,4-tetrahydroisoquinoline-3-carboxylic acid were suspended in 10 ml of dimethylacetamide and 2.5 g of 3-acetylthio-2-acetylthiomethylpropionyl chloride were added dropwise to the solution with ice-cooling and stirring. After stirring at room temperature for 6 hours, the reaction mixture was poured into 200 ml of water and 40 g of sodium chloride were added thereto. The mixture was extracted three times with ethyl acetate (150 ml, 100 ml and 50 ml, respectively), and the combined extract was washed successively with 100 ml of 1N hydrochloric acid and then 100 ml of water. After drying over anhydrous sodium sulfate, the solvent was distilled off. The residue was subjected to column chromatography packed with 100 g of silica gel onto which 1 g of oxalic acid had been adsorbed, and eluted with a mixed solvent of benzene-acetone-methanol (10:10:1). The solvent was distilled off, the residue was dissolved in ethyl acetate and the solution was washed five times with water to remove oxalic acid. The product was dried over anhydrous sodium sulfate and subjected to distillation to remove ethyl acetate, and the residue was extracted several times with diethyl ether. The combined ethereal solution was treated with activated carbon and concentrated. Addition of petroleum ether to the residue yielded pale yellow amorphous powder, filtration of which gave 1.4 g of (S)-2-(3- acetylthio-2-acetylthiomethyl-propionyl)-6,7-dihydroxy-1,2,3,4-tetrahydroisoquinoline-3-carboxylic acid.
Elemental Analysis for C₁₈H₂₁NO₇S₂·1/2H₂O:
   Calcd. : C, 49.52; H, 5.08; N, 3.21
   Found : C, 49.44; H, 4.90; N, 3.12
Mass Spectrum m/e: 427 (M⁺), 384 (M―COCH₃), 208 (M―COCH(CH₂SCOCH₃)₂)
NMR Spectrum (acetone-dₑ) δ:
   2.22 (3H, s, SCOCH ), 2.30 (3H, s, SCOCH₃), 2.6-3.6 (7H, m), 4.1-5.4 (3H, m), 6.68 (2H, s, phenyl proton), 6.9 (2H, br. s, OHx2)
Optical Rotation: 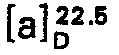 +8.2° (c=0.40, in methanol).

### Example 56

2 g of 7-tert-butyl-1,2,3,4-tetrahydroisoquinoline-3-carboxylic acid were suspended in 30 ml of dimethylacetamide and then 2.2 g of 3-acetylthio-2-acetylthiomethylpropionyl chloride were added dropwise thereto with stirring at room temperature. After stirring at room temperature for 30 minutes, the mixture was subjected to extraction with 300 ml of ethyl acetate and 200 ml of water. The organic layer was washed twice with 200 ml of water, dried over anhydrous sodium sulfate and evaporated under reduced pressure to give a light yellow oily substance. The resulting product was subjected to silica gel column chromatography, eluted with acetone-benzene (1:4 to 1:1) to give 1.8 g of 2-(3- acetylthio-2-acetylthiomethylpropionyl)-7-tert-butyl-1,2,3,4-tetrahydroisoquinoline-3-carboxylic acid as an oily substance, which was obtained as colourless powder upon drying under reduced pressure.
Elemental Analysis for C₂₂H₂₉NO₅S₂:
   Calcd. : C, 58.53; H, 6.47; N, 3.10
   Found : C, 58.17; H, 6.43; N, 3.12
NMR Spectrum (in CDCl₃) δ:
   1.3 (9H, (CH₃)₃―), 2.2-2.4 (6H, CH₃COS), 3.0-3.4 (7H), 4.5-5.4 (3H), 7.0-7.3 (3H, phenyl proton), 8.2 (1 H, COOH).
Mass Spectrum m/3: 451 (M⁺), 408 (M―CH₃CO), 232 (M―COCH(CH₂SCOCH₃)₂)

### Example 57 to 66

The following compounds were obtained as colourless powder by a similar procedure to that of Example 55 or 56.

### Example 57

2-(3-acetylthio-2-acetylthiomethylpropionyl)-1,2,3,4,6,7,8,9-octahydrobenzo[g]isoquinoline-3-carboxylic acid.
Elemental Analysis for C₂₂H₂₇NO₅S₂·H₂O:
   Calcd. : C, 56.52; H, 6.25; N, 3.00
   Found : C, 56.86; H, 6.05; N, 2.88
NMR Spectrum (in CDCI₃) 8:
   1.75 (4H, s), 2.20-2.35 (6H, m, CH₃COS), 2.70 (4H, s), 2.90-3.35 (7H, m), 4.50-4.80 (2H, m), 5.20-5.46 (1H, m), 6.80 (2H, s. phenyl proton), 8.33 (1H, s, COOH).
Mass Spectrum m/e: 449 (M⁺), 406 (M―COCH₃), 230 (M―COCH(CH₂SCOCH₃)₂)

### Example 58

2-(3-acetylthio-2-acetylthiomethylpropionyl)-6,7-methylenedioxy-1,2,3,4-tetrahydroisoquinoline-3-carboxylic acid.
Elemental Analysis for C₁₉H₂₁NO₇S₂·1/2H₂O:
   Calcd. : C, 50.89; H, 4.95; N,3.12
   Found : C, 50,80; H, 4.81; N, 3.07
NMR Spectrum (in CDCl₃) δ:
   2.25 (3H, s, CH₃COS), 2.33 (3H, s, CH₃COS), 2.80-3.40 (7H, m), 4.40-4.95 (2H, m),
   5.20-5.32 (1 H, m), 5.46 (2H, s, ―OCH₂O―), 6.60 (1H, s, phenyl proton), 6.66 (1H, s, phenyl proton), 9.90 (1 H, s, COOH).
Mass Spectrum m/e: 439 (M⁺), 395 (M-COCH₃), 220 (M―COCH(CH₂SCOCH₃)₂)

### Example 59

2-(3-acetylthio-2-acetylthiomethylpropionyl)-6-hydroxy-1,2,3,4-tetrahydroisoquinoline-3-carboxylic acid.
Elemental Analysis for C₁₈H₂₁NO₆S₂·1/2H₂O:
   Calcd. : C, 51.41; H, 5.27; N, 3.33
   Found : C, 51.52; H, 5.06; N, 3.42
NMR Spectrum (in DMSO-d₆) 5:
   2.20 (3H, s, CH₃CO), 2.33 (3H, s, CH₃CO), 2.80-3.40 (7H, m), 4.30-4.70 (2H, m), 4.90-5.20
   (1H, m), 6.50-7.08 (3H, m, phenyl proton), 9.0-9.40 (1H, br. COOH).
Mass Spectrum m/e: 411 (M⁺), 368 (M―COCH₃), 192 (M―COCH(CH₂SCOCH₃)₂)

### Example 60

2-(3-acetylthio-2-acetylthiomethylpropionyl)-7-isopropyl-1,2,3,4-tetrahydroisoquinoline-3-carboxylic acid.
Elemental Analysis for C₂₁H₂₇NO₅S₂·1/2H₂O:
   Calcd. : C, 56.49; H, 6.32; N, 3.14
   Found : C, 56.58; H, 6.45; N, 3.27
NMR Spectrum (in CDCl₃) δ:
   1.20 (6H, d, J=7.5Hz), 2.20 (3H, s, SCOCH₃), 2.33 (3H, s, SCOCH₃), 2.70-3.60 (7H, m),
   4.75-5.40 (3H, m), 7.03-7.60 (3H, m phenyl proton), 7.80 (1H, s, COOH).
Mass Spectrum m/e: 437 (M⁺), 394 (M-COCH₃), 218 (M―COCH(CH₂SCOCH₃)₂)

### Example 61

2-(3-acetylthio-2-acetylthiomethylpropionyl)-1,2,3,4,7,8-hexahydro-6H-cyclopent[g]isoquinoline-3-carboxylic acid.
Elemental Analysis for C₂₁H₂₅NO₅S₂·H₂O:
   Calcd. : C, 55.62; H, 6.00; N, 3.09
   Found : C, 55.42; H, 5.72; N, 3.12
NMR Spectrum (in CDCl₃) 8:
   1.80―210 (2H, m), 2.20 (3H, s, SCOCH₃), 2.30 (3H, s, SCOCH₃), 2.75-3.30 (11H, m),
   4.40-5.30 (3H, m), 7.00 (2H, d, 3Hz phenyl proton), 8.83 (1H, s, COOH).
Mass Spectrum m/e: 435 (M⁺), 392 (M-COCH₃), 216 (M―COCH(CH₂SCOCH₃)₂)

### Example 62

2-(3-acetylthio-2-acetylthiomethylpropionyl)-7-methoxy-1,2,3,4-tetrahydroisoquinoline-3-carboxylic acid.
Elemental Analysis for C₁₉H₂₃NO₆S₂:
   Calcd. : C, 53.64; H, 5.45; N, 3.29
   Found : C, 53.62; H, 6.02; N, 3.06
NMR Spectrum (in CDCl₃) δ:
   2.2 (3H, s, CH₃COS), 2.3 (3H, s, CH₃COS), 2.9―3.4 (7H, m), 3.7 (3H, s, CH₃O), 4.5―5.4 (3H, m), 6.6-7.2 (3H, m, phenyl proton), 8.5 (1 H, s, COOH).
Mass Spectrum m/e: 425 (M⁺), 382 (M-COCH₃), 206 (M―COCH(CH₂SCOCH₃)₂)

### Example 63

2-(3-acetylthio-2-acetylthiomethylpropionyl)-5,6-dimethoxy-1,2,3,4-tetrahydroisoquinoline-3-carboxylic acid.
Elemental Analysis for C₂₀H₂₅SNO₇S₂:
   Calcd. : C, 56.72; H, 5.95; N, 3.31
   Found : C, 56.88; H, 6.03; N, 3.60

### Example 64

(1R,3S)-2-(3-acetylthio-2-acetylthiomethylpropionyl)-6,7-dihydroxy-1-methyl-1,2,3,4-tetrahydroisoquinoline-3-carboxylic acid.
Elemental Analysis for C₁₉H₂₃NO₇S₂.1/2H₂O:
   Calcd. : C, 50.65; H, 5.37; N, 3.11
   Found : C, 50.87; H, 5.36; N, 2.94
Mass Spectrum m/e: 441 (M⁺), 426 (M-CH₃), 222 (M-COCH(CH₂SCOCH₃)₂)
NMR Spectrum (dimethylsulfoxide-d₆) δ:
   1.2 (3H, 1-methyl), 2.2-2.4 (6H, SCOCH₃×2), 2.6-3.7 (7H), 4.6-5.4 (2H, 6.4-6.7 (2H, phenyl proton, 8.7 (2H, OHx2)
Optical Rotation: 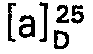 -26.9° (c=0.475, methanol)

### Example 65

2-(3-acetylthio-2-acetylthiomethylpropionyl)-7-cyclopentyl-1,2,3,4-tetrahydroisoquinoline-3-carboxylic acid.
Elemental Analysis for C₂₃H₂₉NO₅S₂:
   Calcd. : C, 59.60; H, 6.31; N, 3.02
   Found : C, 59.27; H, 6.25; N, 2.76
NMR Spectrum (in CDCI ) δ:
   1.50-2.00 (8H, m³), 2.20-2.30 (6H, m, SCOCH₃), 2.80-3.30 (6H, m), 4.65-4.70 (2H, m),
   5.20-5.40 (1H, m), 6.95-7.10 (3H, m, phenyl proton), 8.40 (1H, s, COOH).
Mass Spectrum m/e: 463 (M⁺), 420 (M―COCH₃),

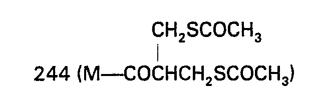

### Example 66

(3S)-(3-acetylthio-2-acetylthiomethylpropionyl)-6,7-methylenedioxy-1,2,3,4-tetrahydroisoquinoline-3-carboxylic acid.
Elemental Analysis for C₁₉H₂₁N0₇S₂:
   Calcd. : C, 51 .91; H, 4.82; N, 3.19
   Found : C, 51.77; H, 4.88; N, 3.18
Optical Rotation: 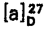 +10.7° (c=0.675, methanol).

### Example 67

In the same manner as described in Example 56 but using ethyl 6,7-methylenedioxy-1,2,3,4-tetrahydroisoquinoline-3-carboxylate as the starting material, ethyl 2-(3-acetylthio-2-acetylthiomethyl- propionyl)-6,7-methylenedioxy-1,2,3,4-tetrahydroisoquinoline-3-carboxylate was obtained.
Elemental Analysis for C₂₁H₂₆NO₇S₂:
   Calcd. : C, 53.96; H, 5.39; N, 3.00
   Found : C, 53.86; H, 5.43; N, 2.94
NMR Spectrum (in CDCl₃) δ:
   1.13 (3H, t, J=6Hz, OCH₂CH₃), 2.25 (3H, s, SCOCH₃), 2.36 (3H, s, SCOCH₃), 3.03―3.50 (7H, m), 4.06 (2H, q, J=6Hz, OCH₂CH₃), 4.05-5.40 (3H, m), 5.80 (2H, s, ―OCH₂O―), 6.60 (1 H, s, phenyl proton), 6.70 (1 H, s, phenyl proton), 7.30 (1 H, s, COOH).
Mass Spectrum m/e: 467 (M⁺), 424 (M―COCH₃), 248 (M―COCH(CH₂SCOCH₃)₂)

### Example 68

2.0 g of 2-(3-acetylthio-2-acetylthiomethylpropionyl)-6,7-methylenedioxy-1,2,3,4-tetrahydroisoquinoline-3-carboxylic acid were dissolved in 20 ml of 5.5N methanolic ammonia, followed by stirring at room temperature for 30 minutes. The reaction solution was concentrated under reduced pressure, 50 ml of water were added to the residue, and, after rendering the mixture acidic with acetic acid, the mixture was extracted with 100 ml of ethyl acetate. The extract was washed with water, dried over anhydrous sodium sulfate, treated with activated carbon and concentrated under reduced pressure to give 1.2 g of 2-(3-mercapto-2-mercaptomethylpropionyl)-6,7-methylenedioxy-1,2,3,4-tetrahydroisoquinoline-3-carboxylic acid as an oily substance. The product turned into a colourless powder upon drying under reduced pressure.
Elemental Analysis for C₁₅H₁₇NO₅S₂.1/2H₂O:
   Calcd. : C, 49.45; H, 4.98; N, 3.85
   Found : C, 49.28; H, 4.95; N, 4.07
NMR Spectrum (in CDCI₃) δ:
   2.50-3.45 (7H, m), 4.75 (2H, s, CH₂), 5.30-5.40 (1 H, m), 5.90 (2H, s, -OCH₂0-), 6.60 (1 H, s, phenyl proton), 6.63 (1 H, s, phenyl proton), 7.95 (1 H, s, COOH).
Mass Spectrum m/e: 355 (M⁺), 220 (M―COCH(CH₂SH)₂)

### Examples 69 to 72

In the same manner as described in Example 50 but, using the compounds obtained in Examples 55, 56, 57 and 66 as the respective starting materials, the compounds of Examples 69 to 72 were obtained.

### Example 69

(S)-6,7-dihydroxy-2-(3-mercapto-2-mercaptomethylpropionyl)-1,2,3,4-tetrahydroisoquinoline-3-carboxylic acid.
Elemental Analysis for C₁₄H₁₇NO₅S₂:
   Calcd. : C, 48.98; H, 4.99; N, 4.08
   Found : C, 48.68; H, 5.25; N, 4.37
Mass Spectrum m/e: 343 (M⁺), 298 (M-COOH), 208 (M-COCH(CH₂SH)₂)

### Example 70

7-tert-butyl-2-(3-mercapto-2-mercaptomethylprionyl)-1,2,3,4-tetrahydroisoquinoline-3-carboxylic acid.
Elemental Analysis for C₁₈H₂₅NO₃S₂:
   Calcd. : C, 58.84; H, 6.86; N, 3.81
   Found : C, 58.51; H, 7.03; N, 3.66
NMR Spectrum (in CDCl₃―D₂O) δ:
   1.3 (9H, s, (CH₃)₃C), 2.2-3.4 (7H, m), 4.6-5.5 (3H), 7.0-7.3 (3H, m),
Mass Spectrum m/e: 367 (M⁺), 232 (M―COCH(CH₂SH)₂)

### Example 71

2-(3-mercapto-2-mercaptomethylpropionyl)-1,2,3,4,6,7,8,9-octahydrobenzo[g]isoquinoline-3-carboxylic acid.
Elemental Analysis for C₁₈H₂₃NO₃S2·1/2H₂O:
   Calcd. : C, 57.74; H, 6.46; N, 3.74
   Found : C, 57.79; H, 6.28; N, 3.85
NMR Spectrum (in CDCl₃) 8:
   1.70 (1 H, s,), 2.76-3.40 (1 H, m), 4.80 (2H, s), 5.33 (1 H, t, J=6Hz), 6.80 (2H, d, J=3Hz, phenyl proton), 8.33 (1 H, s, COOH).
Mass Spectrum m/e: 365 (M⁺), 230 (M-COCH(CH₂SH)₂)

### Example 72

(3S)-2-(3-mercapto-2-mercaptomethylpropionyl)-6,7-methylenedioxy-1,2,3,4-tetrahydroisoquinoline-3-carboxylic acid.
Elemental Analysis for C₁₅H₁₇NO₅S₂:
   Calcd. : C, 50.71; H, 4.82; N, 3.94
   Found : C, 50.99; H, 4.76; N, 3.74
Optical Rotation: 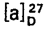 +8.8° (c=0.74, methanol)

### Example 73

1 g of 2-(3-mercapto-2-mercaptomethylpropionyl)-6,7-methylenedioxy-1,2,3,4-tetrahydroisoquinoline-3-carboxylic acid was dissolved in 20 ml of ethanol and 5% ethanolic solution of iodine was added dropwise thereto with stirring under ice-cooling. The addition was continued until the iodine colour no longer disappeared. The reaction mixture was poured into 100 ml of water, and extracted with ethyl acetate. After drying the extract over anhydrous sodium sulfate, ethyl acetate was distilled off under reduced pressure at a temperature below 40°C, and the residue was dissolved in diethyl ether. Insoluble materials were filtered off with a small amount of activated carbon, and diethyl ether was distilled off under reduced pressure at room temperature to give 0.2 g of 2-(1,2-dithiolan-4-carbonyl)-6.7-rnethylenedioxy-1,2,3,4-tetrahydroisoquinoline-3-carboxylic acid as light yellow syrup.
Elemental Analysis for C₁₅H₁₅NO₅S₂:
   Calcd. : C, 50.99; H, 4.28; N, 3.97
   Found : C, 51.03; H, 4.39; N, 4.08
NMR Spectrum (in CDC1₃) 8:
   2.45-3.50 (7H, m), 4.78 (2H, s, CH₂), 5.30-5.40 (1 H, m), 5.95 (2H, s, -OCH₂0-), 6.60 (1 H, s, phenyl proton), 6.65 (1 H, s, phenyl proton), 7.95 (1 H, s, COOH).
Mass Spectrum m/d: 353 (M⁺),

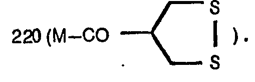

### Experiment 1

Inhibitions of Angiotensin I Converting Enzyme by the Compounds of this Invention.

### Experimental Method

The experiment was conducted in accordance with a modification of the method described by Cushman et al. [Biochemical Pharmacology, Vol. 20, p 1637 (1971)] using an angiotensin I converting enzyme (ACE) prepared from the lungs of albino rabbit according to the method described by Wallace et al. [American Journal of Physiology, Vol. 234, page R141 (1978)]. That is, using hippuryl-L-histidyl-L-leucin (HHL) as the substrate, the ACE inhibitory activity was determined in terms of percentage inhibitation on the amount of hippuric acid produced by ACE when the present compound was added. A solution of the compound of the present invention dissolved in an 0.02 to 2% dimethyl sulfoxide-500 mM potassium phosphate buffer solution (pH 8.3, containing 300 mM sodium chloride) was added to 100 µl of ACE (protein concentration, 20 mg/ml) and 100 ul of 1.25 mM HHL. In this experiment, a potassium phosphate buffer solution containing dimethyl sulfoxide at a concentration equal to that of the test solution was used as a control. After incubating the solution at 37°C for one hour, 150 µl of 1 N hydrochloric acid were added to the solution to terminate the reaction. After 1 ml of ethyl acetate was added, the solution was centrifuged at 3000 r.p.m. for 10 minutes. An 0.5 ml aliquot was separated from the ethyl acetate layer and dried at a temperature below 50°C under a nitrogen gas stream. The residue was mixed with 5 ml of 1 M aqueous sodium chloride and the mixture was subjected to colorimetry at a wavelength of 228 nm.

### Test Results

The test results obtained with respect to the compounds of Examples 1, 6, 7, 14, 19, 34, 38, 42, 44, 52 and 70 are shown in Table 3 below.

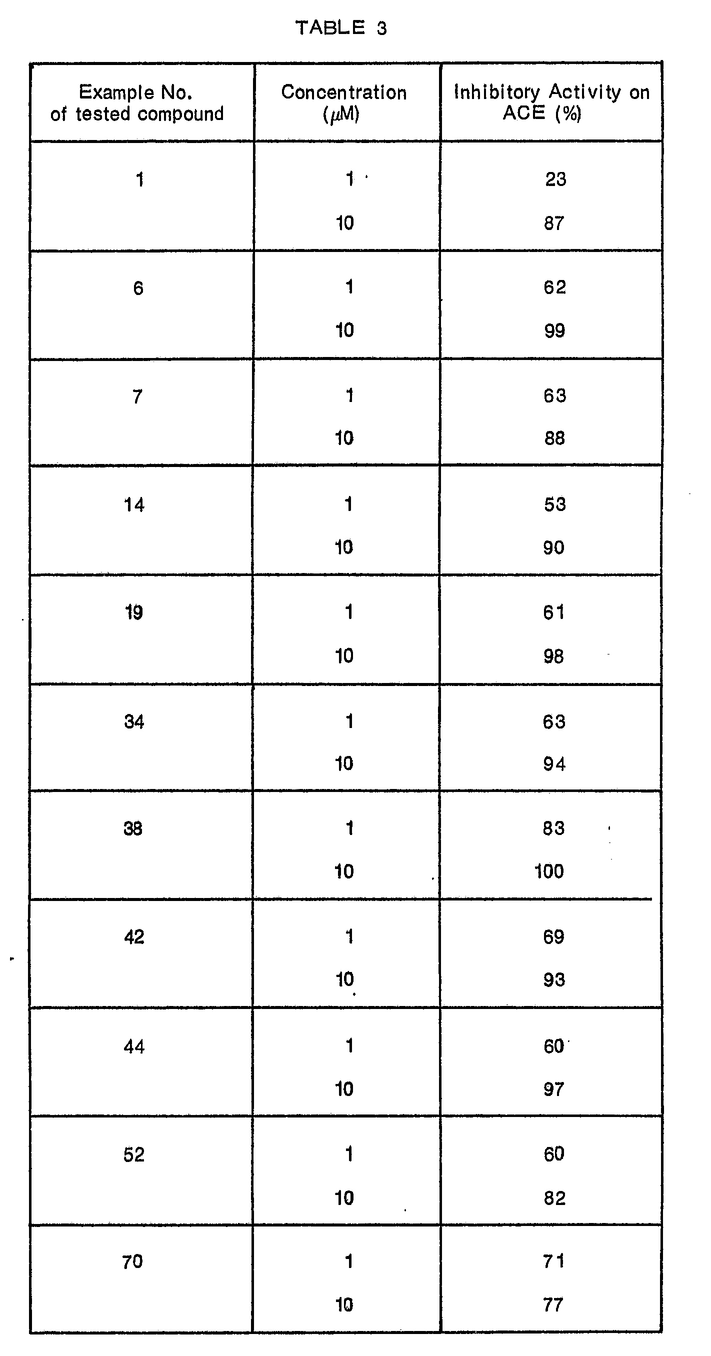

### Experiment Example 2

### Effect of Present Compounds against Hypertensive Activity of Angiotensin I

### Experiment Method

Male rats (Sprague-Dawley) weighing 250 to 350 g, which were fed under free access to drinking water and feeds, were used as experimental animals. The rats were anaesthetized with intraperitoneal administration of pentobarbital sodium (50 mg/kg) on the day before the test day, and a polyethylene tube was inserted into each femoral artery for measurement of blood pressure and the femoral vein for injection of angiotensin I and II, and then the tubes were fixed.

On the test day, an average blood pressure in the control phase was recorded on an electric haemodynamometer (MP--4T model manufactured by Nippon Koden, Japan) and, thereafter, angiotensin I and then angiotensin II were injected through the femoral vein at a dose of 300 ng/kg and 100 ng/kg, respectively, to measure the hypertensive activity. Then, 138 µM/kg of the compound of this invention were administered orally as an aqueous solution or an aqueous gum arabic suspension, and 20, 60 and 120 minutes after the administration, angiotensin I and II were injected repeatedly to trace hypertensive reactions. In calculating the percentage inhibition to the hypertensive activity of angiotensin I, the percentage inhibitory value was corrected based on the variation with time in the hypertensive reaction by angiotensin II.

### Test Results

The test results obtained with respect to the compounds of Examples 3, 4, 5, 23, 43, 51, 55, 57, 59, 60, 68 are shown in Table 4 below.

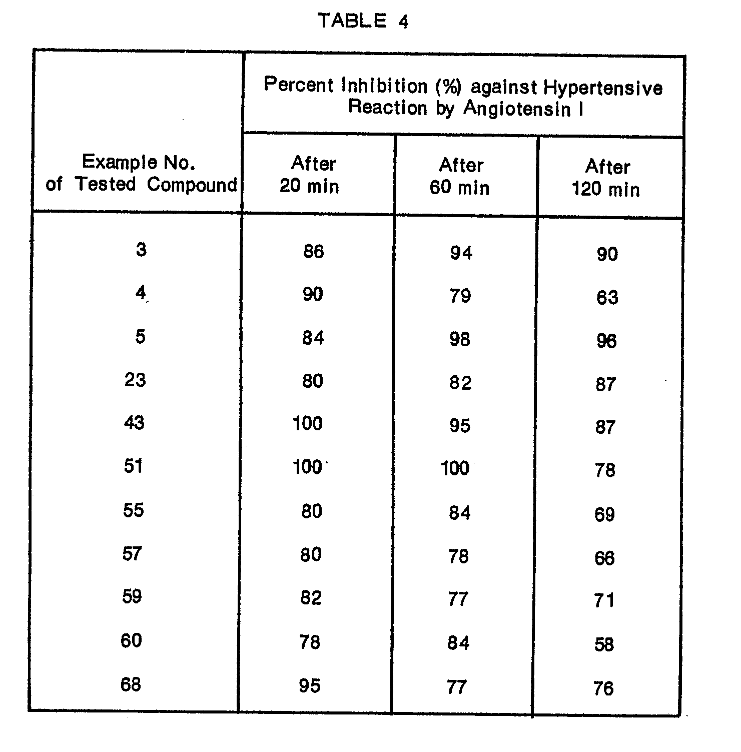

### Preparation Example

The compounds (I) of the present invention are used, for example, for the treatment of hypertension in the following examples of formulation.

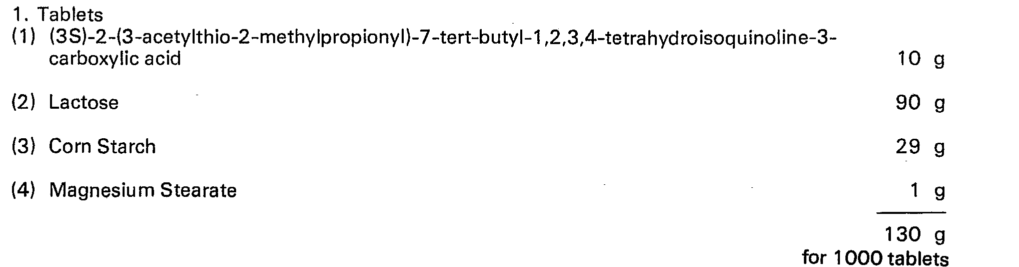

The above ingredients (1) and (2) and 17 g of corn starch were blended, and granulated using a paste prepared from 7 g of corn starch. 5 g of corn starch and the ingredient (4) were added to the resulting granules and the mixture was compressed by a tabletting machine to prepare 1000 tablets having a diameter of 7 mm, each containing 10 mg of the active ingredient (1).

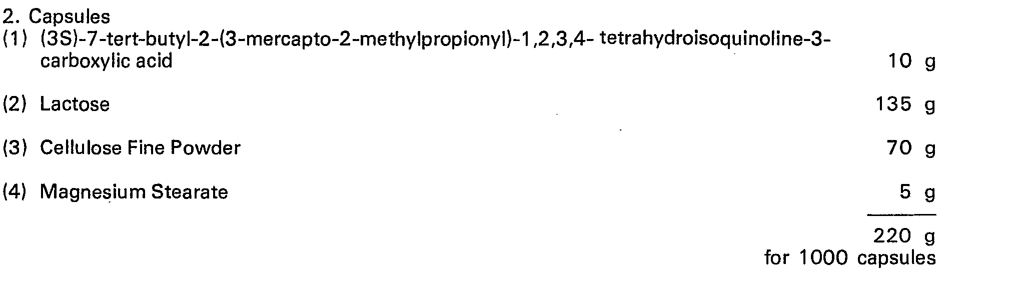

All of the above components were blended and encapsulated into Gelatin Capsule No. 3 (IX Japanese Pharmacopoiea) to prepare 1000 capsules each containing 10 mg of the active component (1).

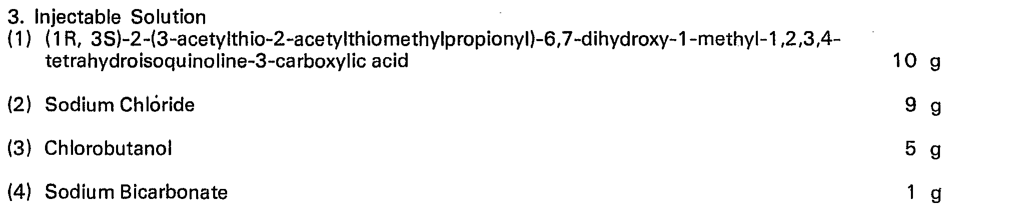

All of the above ingredients are dissolved in 1000 ml of distilled water and charged into 1000 brown ampoules each containing 1 ml of the solution. The ampoules were purified with gaseous nitrogen and sealed. The entire preparation steps were conducted under sterile conditions.

## Claims (Claims for the following Contracting State(s) : s: BE CH DE FR GB IT LU NL SE)

1. A compound of the formula (I): 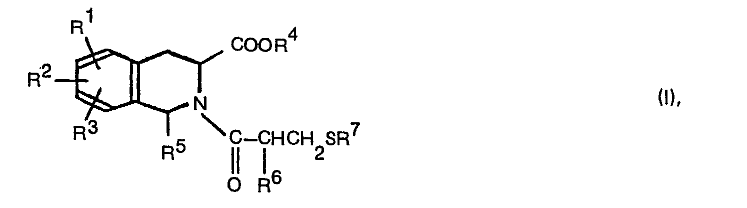 wherein
R¹, R² and R³, which may be the same or different, each represent hydrogen, halogen, nitro, hydroxyl, C₁₋₄ alkyl, C₅₋₇ cycloalkyl, C₁₋₄ alkoxy or 5 to 7 membered cyclic amino, selected from pyrrolidinyl, piperidino, homopiperidinyl, piperazinyl and morpholino, with the proviso that, when both R¹ and R² are hydrogen, R³ is one of the substituents defined above except hydrogen, or any two adjacent groups R', R² and R³ form C₁₋₄ alkylenedioxy or C₃₋₆ alkylene,
R⁴ and R⁵ each represent hydrogen or C₁₋₄ alkyl,
R⁶ is hydrogen, C₁₋₄ alkyl or -CH₂SR⁸ in which
R⁸ is hydrogen or C₂₋₄ alkanoyl, and
R⁷ is hydrogen, C₂₋₄ alkanoyl or benzoyl, or
R⁷ and R⁸ jointly represent a single bond when R⁶ is CH₂SR⁸,
or a pharmaceutically acceptable salt thereof.

2. A compound according to claim 1, wherein R¹, R² and R³, which may be the same or different, each represent hydrogen, hydroxyl, C₁₋₄ alkyl or C₁₋₄ alkoxy, provided that, when both R¹ and R² are hydrogen, R³ is one of the substituents defined above except hydrogen, or any two adjacent groups of R¹, R² and R³ form C₁₋₄ alkylenedioxy.

3. A compound according to claim 2, wherein R' is hydrogen and R² and R³ are hydroxyl or C₁₋₄ alkoxy at the 6- and 7-positions, or R² together with R³ represents methylenedioxy at the 6- and 7- positions.

4. A compound according to claim 2, wherein R¹ and R² are hydrogen and R³ is C₁₋₄ alkyl at the 7- position.

5. A compound according to any of claims 1 to 4, wherein R⁴ is hydrogen, R⁵ is hydrogen or methyl, R⁶ is hydrogen, methyl or ―CH₂SR⁸ in which R⁸ is hydrogen or acetyl, and R⁷ is hydrogen or acetyl, or R⁷ and R⁸ jointly represent a single bond when R^{S} is -CH₂SR⁸.

6. The compound according to claim 1, which is 2-(3-acetylthio-2-methylpropionyl)-7-tert-butyl-1,2,3,4-tetrahydroisoquinoline-3-carboxylic acid.

7. The compound according to claim 1, which is 7-tert-butyl-2-(3-mercapto-2-methylpropionyl)-1,2,3,4-tetrahydroisoquinoline-3-carboxylic acid.

8. The compound according to claim 1, which is 2-(3-acetylthio-2-acetylthiomethylpropionyl)-7-tert-butyl-1,2,3,4-tetrahydroisoquinoline-3-carboxylic acid.

9. The compound according to claim 1, which is 2-(3-acetylthio-2-methylpropionyl)-7-isopropyl-1,2,3,4-tetrahydroisoquinoline-3-carboxylic acid.

10. The compound according to claim 1, which is 2-(3-acetylthio-2-acetylthiomethylpropionyl)-8,7-dihydroxy-1-methyl-1,2,3,4-tetrahydroisoquinoline-3-carboxylic acid.

11. The compound according to claim 1, which is 2-(3-mercapto-2-mercaptomethylpropionyl)-8,7-methylenedioxy-1,2,3,4-tetrahydroisoquinoline-3-carboxylic acid.

12. A pharmaceutical composition which comprises, as an active ingredient, an effective amount of a compound as defined in any of claims 1-11, or a pharmaceutically acceptable salt thereof, and a pharmaceutically acceptable carrier, excipient or diluent therefor.

13. A compound of the formula (I) as defined in any of claims .1-11, or a pharmaceutically acceptable salt thereof, or a pharmaceutical composition as claimed in claim 12, for use in the prevention or treatment of hypertension in a mammal.

14. A method of producing a compound of the formula (I) as defined in claim 1, or a pharmaceutically acceptable salt thereof, which comprises reacting a compound of the formula (II): 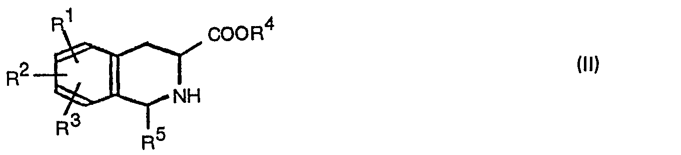 wherein all the symbols are as defined above, with a compound of the formula (III): 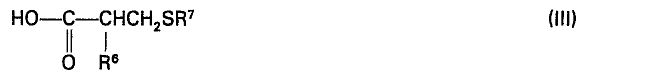 wherein R⁸ and R⁷ are as defined above, or its reactive derivative at the carboxyl function, and optionally converting said compound of the formula (I) to the pharmaceutically acceptable salt.

## Claims (Claims for the following Contracting State(s) : AT)

1. A method of producing a compound of the formula (I): 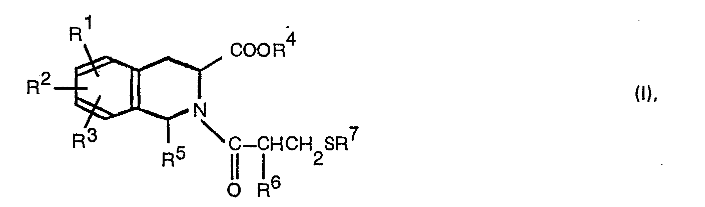 wherein
R¹, R² and R³, which may be the same or different, each represent hydrogen, halogen, nitro, hydroxyl, C₁₋₄ alkyl, C₅₋₇ cyloalkyl, C₁₋₄ alkoxy or 5 to 7 membered cyclic amino selected from pyrrolidinyl, piperidino, homopiperidinyl, piperazinyl and morpholino, with the proviso that, when both R¹ and R² are hydrogen, R³ is one of the substituents defined above except hydrogen, or any two of the adjacent groups R¹, R² and R³ form C₁₋₄ alkylenedioxy or C₃₋₆ alkylene,
R⁴ and R⁵ each represent hydrogen or C₁₋₄ alkyl,
R⁶ is hydrogen, C₁₋₄ alkyl or ―CH₂SR⁸ in which
R⁸ is hydrogen or C₂₋₄ alkanoyl, and
R⁷ is hydrogen or C₂₋₄ alkanoyl or benzoyl, or
R⁷ and R⁸ jointly represent a single bond when R⁵ is ―CH₂SR⁸,
or a pharmaceutically acceptable salt thereof, which comprises reacting a compound of the formula (II): 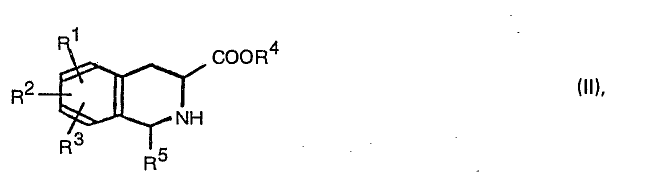 wherein all the symbols are as defined above, with a compound of the formula (III): 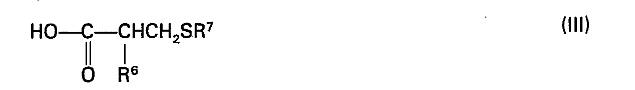 wherein R⁶ and R⁷ are as defined above, or its reactive derivative at the carboxyl function, and optionally converting said compound of the formula (I) to its pharmaceutically acceptable salt.

2. A method according to claim 1, wherein R¹, R² and R³, which may be the same or different, each represent hydrogen, hydroxyl, C₁₋₄ alkyl or C₁₋₄ alkoxy, provided that, when both R¹ and R² are hydrogen, R³ is one of the substituents defined above except hydrogen, or any two adjacent groups R¹, R² and R³ form C₁₋₄ alkylenedioxy

3. A method according to claim 2, wherein R¹ is hydrogen and R² and R³ are hydroxyl or C₁₋₄ alkoxy at the 6- and 7-positions, or R² together with R³ represents methylenedioxy at the 6- and 7- positions.

4. A method according to claim 2, wherein R¹ and R² are hydrogen and R³ is C₁₋₄ alkyl at the 7- position.

5. A method according to any of claims 1 to 4, wherein R⁴ is hydrogen, R⁵ is hydrogen or methyl, R⁸ is hydrogen, methyl or ―CH₂SR⁸ in which R⁸ is hydrogen or acetyl, and R⁷ is hydrogen or acetyl, or R⁷ and R⁸ jointly represent a single bond when R⁶ is ―CH₂SR⁸.

6. A method according to claim 1, wherein said compound of the formula (I) is 2-(3-acetylthio-2-methylpropionyl)-7-tert-butyl-1,2,3,4-tetrahydroisoquinoline-3-carboxylic acid.

7. A method according to claim 1, wherein said compound of the formula (I) is 7-tert-butyl-2-(3-mercapto-2-methylpropionyl)-1,2,3,4-tetra-hydroisoquinoline-3-carboxylic acid.

8. A method according to claim 1, wherein said compound of the formula (I) is 2-(3-acetylthio-2- acetylthiomethylpropionyl)-7-tert-butyl-1,2,3,4-tetrahydroisoquinoline-3-carboxylic acid.

9. A method according to claim 1, wherein said compound of the formula (I) is 2-(3-acetylthio-2-methylpropionyl)-7-isopropyl-1,2,3,4-tetrahydroisoquinoline-3-carboxylic acid.

10. A method according to claim 1, wherein said compound of the formula (I) is 2-(3-acetylthio-2-acetylthiomethylpropionyl)-6,7-dihydroxy-1-methyl-1,2,3,4-tetrahydroisoquinoline-3-carboxylic acid.

11. A method according to claim 1, wherein said compound of the formula (I) is 2-(3-mercapto-2-mercaptomethylpropionyl)-6,7-methylenedioxy-1,2,3,4-tetrahydroisoquinoline-3-carboxylic acid.

12. A compound of the formula (I) as defined in any of claims 1 to 11, or a pharmaceutically acceptable salt thereof, or a pharmaceutical composition which comprises, as an active ingredient, an effective amount of a compound of the formula (I) as defined in any of claims 1 to 11, or a pharmaceutically acceptable salt thereof, together with a pharmaceutically acceptable carrier, excipient or diluent therefor, for use in the prevention or treatment of hypertension in a mammal.

## Revendications (Revendications pour l'(les) Etat(s) contractant(s) suivant(s) : BE CH DE FR GB IT LU NL SE)

1. Composé ayant la formule (I): 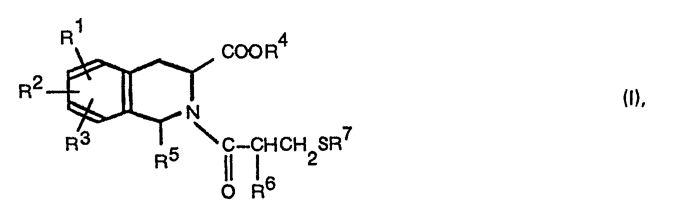 dans laquelle
R¹, R² et R³, qui peuvent être identiques ou différents, représentent chacun de l'hydrogène, de l'halogène, un groupe nitro, hydroxyle, C₁₋₄alkyle, C₅₋₇cycloalkyle, C₁₋₄alcoxy ou un groupe amino- cyclique de 5 à 7 chaînons, choisi parmi les groupes pyrrolidinyle, pipéridino, homopipéridinyle, pipérazinyle et morpholino, avec la condition que lorsque R¹ et R² sont tous deux de l'hydrogène, R soit un des substituants définis ci-dessus, sauf de l'hydrogène, ou bien deux quelconques des groupes voisins R¹, R² et R³ forment un groupe C₁₋₄alkylènedioxy ou C₃₋₆alkylène;
R⁴ et R⁵ représentent chacun de l'hydrogène ou un groupe C₁₋₄alkyle;
R⁴ est de l'hydrogène, un groupe C₁₋₄alkyle ou -CH₂SR⁸ dans lequel R⁸ est de l'hydrogène ou un groupe C₂₋₄alcanoyle, et R⁷ est de l'hydrogène, un groupe C₂₋₄alcanoyle ou benzoyle, ou bien R⁷ et R⁸ représentent conjointement une simple liaison quand R^{S} est CH₂SR⁸,
ou sel de ce composé pharmaceutiquement acceptable.

2. Composé selon la revendication 1, dans lequel R¹, R² et R³, qui peuvent être identiques ou différents, représentent chacun de l'hydrogène, un groupe hydroxyle, C₁₋₄alkyle ou C₁₋₄alcoxy, pourvu que, si R¹ et R² sont tous deux de l'hydrogène, R³ soit un des substituants définis ci-dessus, sauf de l'hydrogène, ou bien deux quelconques des groupes voisins R¹, R² et R³ forment un groupe alkylènedioxy.

3. Composé selon la revendication 2, dans lequel R' est de l'hydrogène, et R² et R³ sont des groupes hydroxyle ou C₁₋₄alcoxy sur les positions 6 et 7, ou bien R² conjointement à R³ représente le groupe méthylènedioxy sur les positions 6 et 7.

4. Composé selon la revendication 2, dans lequel R¹ et R² sont de l'hydrogène, et R³ est un groupe C₁₋₄alkyle sur la position 7.

5. Composé selon l'une quelconque des revendications 1 à 4, dans lequel R⁴ est de l'hydrogène; R⁶ est de l'hydrogène ou le groupe méthyle; R⁶ est de l'hydrogène ou le groupe méthyle ou ―CH₂SR⁸ dans lequel R⁸ est de l'hydrogène ou le groupe acétyle, et R⁷ est de l'hydrogène ou le groupe acétyle, ou bien R⁷ et R⁸ représentent conjointement une simple liaison quand R⁸ est ―CH₂SR⁸.

6. Composé selon la revendication 1, qui est l'acide 2-(3-acétylthio-2-méthylpropionyl)-7-tert-butyl-1,2,3,4-tétrahydro-isoquinoléine-3-carboxylique.

7. Composé selon la revendication 1, qui est l'acide 7-tert-butyl-2-(3-mercapto-2-méthylpropionyl)-1,2,3,4-tétrahydro-isoquinoléine-3-carboxylique.

8. Composé selon la revendication 1, qui est l'acide 2-(3-acétylthio-2-acétylthiométhyl- propionyl)-7-tert-butyl-1,2,3,4-tétrahydro-isoquinoléine-3-carboxylique.

9. Composé selon la revendication 1, qui est l'acide 2-(3-acétylthio-2-méthylpropionyl)-7-isopropyl-1,2,3,4-tétrahydro-isoquinoléine-3-carboxylique.

10. Composé selon la revendication 1, qui est l'acide 2-(3-acétylthio-2-acétylthiométhyl- propionyl)-6,7-dihydroxy-1-méthyl-1,2,3,4-tétrahydro-isoquinoléine-3-carboxylique.

11. Composé selon la revendication 1, qui est l'acide 2-(3-mercapto-2-mercaptométhyl- propionyl)-6,7-méthylènedioxy-1,2,3,4-tétrahydro-isoquinoléine-3-carboxylique.

12. Composition pharmaceutique qui comprend, comme ingrédient actif, une quantité efficace d'un composé tel que défini dans l'une quelconque des revendications 1 à 11, ou d'un sel de ce composé pharmaceutiquement acceptable, un véhicule pharmaceutiquement acceptable, un excipient. ou un diluant pour ces produits.

13. Composé ayant la formule (I) tel que défini dans l'une quelconque des revendications 1 à 11, ou sel de ce composé pharmaceutiquement acceptable, ou composition pharmaceutique telle que revendiquée dans la revendication 12, pour usage dans la prévention ou le traitement de l'hypertension chez les mammifères.

14. Procédé de préparation d'un composé ayant la formule (I) tel que défini dans la revendication 1, ou d'un sel de ce composé pharmaceutiquement acceptable, qui consiste à faire réagir un composé ayant la formule (II): 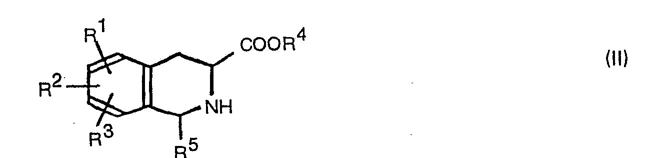 dans laquelle tous les symboles sont tels que définis ci-dessus, avec un composé ayant la formule (III): 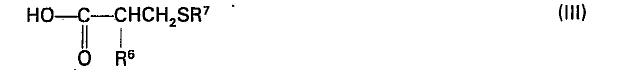 dans laquelle R⁶ et R⁷ sont tels que définis ci-dessus, ou son dérivé réactif sur la fonction carboxyle, et éventuellement à convertir ledit composé ayant la formule (I) en sel pharmaceutiquement acceptable.

## Revendications (Revendications pour l'(les) Etat(s) contractant(s) suivant(s) : AT)

1. Procédé de production d'un composé ayant la formule (I): 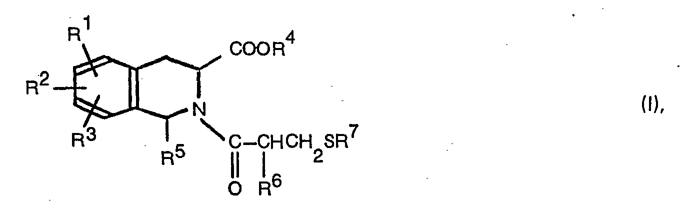 dans laquelle
R¹, R² et R³, qui peuvent être identiques ou différents, représentent chacun de l'hydrogène, de l'halogène, un groupe nitro, hydroxyle, C₁₋₄alkyle, C₅₋₇cycloalkyle, C₁₋₄alcoxy ou un groupe amino cyclique de 5 à 7 chainons, choisi parmi les groupes pyrrolidinyle, pipéridino, homopipéridinyle, pipérazinyle et morpholino, avec la condition que lorsque R¹ et R² sont tous deux de l'hydrogène, R³ désigne un des substituants définis ci-dessus sauf de l'hydrogène, ou bien deux quelconques des groupes voisins R¹, R² et R³ forment un groupe C₁₋₄alkylènedioxy ou C₃₋₆alkylène;
R⁴ et R⁵ représentent chacun de l'hydrogène ou un groupe C₁₋₄alkyle;
R⁶ est de l'hydrogène, un groupe C₁₋₄alkyle ou ―CH₂SR⁸ dans lequel R⁸ est de l'hydrogène ou un groupe C₂₋₄alcanoyle, et R⁷ est de l'hydrogène, un groupe C₂₋₄alcanoyle ou benzoyle, ou bien R⁷ et R^{B} représentent conjointement une simple liaison quand R^{S} est CH₂SR⁸,
ou d'un sel de ce composé pharmaceutiquement acceptable, comprenant la réaction d'un composé de formule (II): 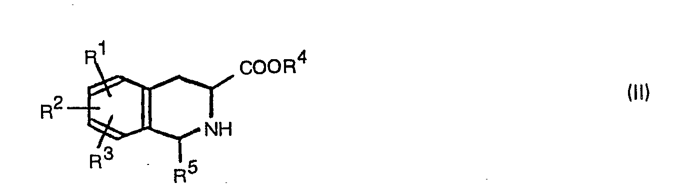 dans laquelle tous les symboles sont comme définis ci-dessus, avec un composé de formule (III): 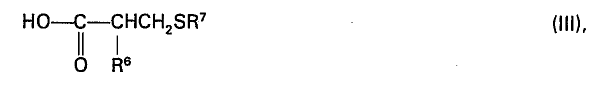 où R₆ et R⁷ sont comme définis ci-dessus, ou son dérivé réactif de la fonction carboxyle et si l'un désire la transformation dudit composé de formule (I) en son sel pharmaceutiquement acceptable.

2. Procédé selon la revendication 1, dans lequel R¹, R² et R³, qui peuvent être identiques ou différents, représentent chacun de l'hydrogène, hydroxyle, C₁₋₄alkyle ou C₁₋₄alcoxy, à condition que, lorsque R¹ et R² sont tous deux de l'hydrogène, R³ désigne un des substituants définis ci-dessus sauf de l'hydrogène, ou bien deux quelconques des groupes voisins R¹, R² et R³ forment un groupe alkylènedioxy.

3. Procédé selon la revendication 2, dans lequel R¹ est de l'hydrogène, et R² et R³ sont hydroxyle ou C₁₋₄alcoxy en positions 6 et 7, ou bien R² conjointement avec R³ représente le groupe méthylènedioxy en positions 6 et 7.

4. Procédé selon la revendication 2, dans lequel R' et R² sont de l'hydrogène, et R³ est un groupe C₁₋₄alkyle en position 7.

5. Procédé selon l'une quelconque des revendications 1 à 4, dans lequel R⁴ est de l'hydrogène; R⁵ est de l'hydrogène ou méthyle; R⁶ est de l'hydrogène ou méthyle ou ―CH₂SR⁸ où R⁸ est de l'hydrogène ou acétyle, et R⁷ est de l'hydrogene ou acétyle, ou bien R⁷ et R⁸ représentent conjointement une liaison simple quand R⁶ est ―CH₂SR⁸.

6. Procédé selon la revendication 1, où ledit composé de formule (I) est l'acide 2-(3-acétylthio-2- méthyipropionyl)-7-tert-butyl-1,2,3,4-tétrahydro-isoquinoléine-3-carboxylique.

7. Procédé selon la revendication 1, où ledit composé de formule (1) est l'acide 7-tert-butyl-2-(3-mercapto-2-méthylpropionyl)-1,2,3,4-tétrahydro-isoquinoléine-3-carboxylique.

8. Procédé selon la revendication 1, où ledit composé de formule (I) est l'acide 2-(3-acétylthio-2-acétylthiométhylpropionyl)-7-tert-butyl-1,2,3,4-tétrahydro-isoquinoléine-3-carboxylique.

9. Procédé selon la revendication 1, où ledit composé de formule (I) est l'acide 2-(3-acétylthio-2-méthylpropionyl)-7-isopropyl-1,2,3,4-tétrahydro-isoquinoléine-3-carboxylique.

10. Procédé selon la revendication 1, où ledit composé de formule (I) est l'acide 2-(3-acétylthio-2-acétylthiométhylpropionyl)-8,7-dihydroxy-1-methyl-1,2,3,4-tétrahydroisoquinoléine-3-carboxylique.

11.Procédé selon la revendication 1, où ledit composé de formule (I) est l'acide 2-(3-mercapto-2-mercaptométhylpropionyl)-6,7-méthylénedioxy-1,2,3,4-tétrahydroisoquinoléine-3-carboxylique.

12. Composé de formule (1), tel que défini dans l'une quelconque des revendications 1 à 11 ou un de ses sels pharmaceutiquement acceptable, une composition pharmaceutique qui comprend, comme Ingrédient actif, une quantité efficace d'un composé de formule (I) tel que défini dans l'une quelconque des revendications 1 à 11 ou un sel pharmaceutiquement acceptable de ce composé, avec un véhicule pharmaceutiquement acceptable, un excipient ou un diluant pour ces produits pour usage dans la prévention ou le traitement de l'hypertension chez les mammifères.

## Patentansprüche (Patentansprüche für folgende(n) Vertragsstaat(en) : BE CH DE FR GB IT LU NL SE)

1. Verbindung der Formel (I) 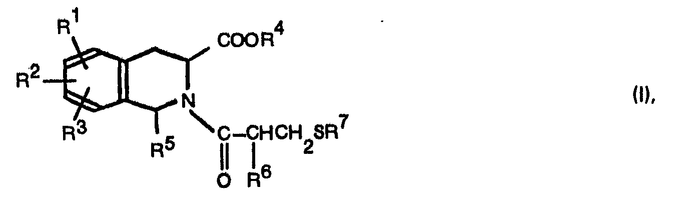 In der
R¹, R² und R³ gleich oder verschieden sein können und jeweils Wasserstoff, Halogen, Nitro, Hydroxyl, C₁- bis C₄-Alkyl, C₅- bis C₇-cycloalkyl, C₁- bis C₄-Alkoxy oder 5- bis 7-gliedriges cyclisches Amino ausgewählt aus Pyrrolidinyl, Piperidino, Homopiperidinyl, Piperazinyl und Morpholino bezeichnen, mit der Maßgabe, daß, wenn R¹ und R² beide Wasserstoff sind, R³ einer der angegebenen, von Wasserstoff verschiedenen Substituenten ist, oder zwei beliebige, einander benachbarte Gruppen R¹, R² und R³ C₁- bis C₄-Alkylendioxy oder C₃- bis C -Alkylen bilden,
R⁴ und R" jeweils Wasserstoff oder C₁- bis C₄-Alkyl bezeichnen,
R⁶ Wasserstoff, C₁- bis C₄-Alkyl oder ―CH₂SR⁸ ist, worin R⁸ Wasserstoff oder C₂- bis C₄-Alkanoyl ist, und
R⁷ Wasserstoff, C₂- bis C₄-Alkanoyl oder Benzoyl ist, oder, wenn R⁶ ―CH₂SR⁸ ist, R⁷ und R⁸ gemeinsam eine Einfach-Bindung bezeichnen,
oder ein pharmazeutisch unbedenkliches Salz derselben.

2. Verbindung nach Anspruch 1, dadurch gekennzeichnet, daß R¹, R² und R³ gleich oder verschieden sein können und jeweils Wasserstoff, Hydroxyl, C₁- bis C₄-Alkyl oder C₁- bis C₄₋Alkoxy bezeichnen, mit der Maßgabe, daß, wenn R' und R² beide Wasserstoff sind, R³ einer der angegebenen, von Wasserstoff verschiedenen Substituenten ist, oder zwei beliebige, einander benachbarte Gruppen R¹, R² und R³ C₁- bis C₄-Alkylendioxy bilden.

3. Verbindung nach Anspruch 2, dadurch gekennzeichnet, daß R¹ Wasserstoff ist und R² und R³ Hydroxyl oder C,- bis C₄₋Alkoxy in den 6- und 7-Stellungen sind oder R² und R³ zusammen Methylendioxy in den 6- und 7-Stellungen bezeichnen.

4. Verbindung nach Anspruch 2, dadurch gekennzeichnet, daß R¹ und R² Wasserstoff sind und R³ C,- bis C₄₋Alkyl in der 7-Stellung ist.

5. Verbindung nach Anspruch 1 bis 4, dadurch gekennzeichnet, daß R⁴ Wasserstoff ist, R⁵ Wasserstoff oder Methyl ist, R⁶ Wasserstoff, Methyl oder ―CH₂SR⁸ ist, worin R⁸ Wasserstoff oder Acetyl ist, und R⁷ Wasserstoff oder Acetyl ist, oder, wenn R⁸ ―CH₂SR⁸ ist, R⁷ und R⁸ gemeinsam eine Einfach-Bindung bezeichnen.

6. Verbindung 2-(3-Acetylthio-2-methylpropionyl)-7-tert-butyl-1,2,3,4-tetrahydroisochinolin-3-carbonsäure nach Anspruch 1.

7. Verbindung 7-tert-Butyl-2-(3-mercapto-2-methylpropionyl)-1,2,3,4-tetrahydroisochinolin-3-carbonsäure nach Anspruch 1.

8. Verbindung 2-(3-Acetylthio-2-acetylthiomethylpropionyl)-7-tert-butyl-1,2,3,4-tetrahydroisochinolin-3-carbonsäure nach Anspruch 1.

9. Verbindung 2-(3-Acetylthio-2-methylpropionyl)-7-isopropyl-1,2,3,4-tetrahydroisochinolin-3-carbonsäure nach Anspruch 1.

10. Verbindung 2-(3-Acetylthio-2-acetylthiomethylpropionyl)-6,7-dihydroxy-1-methyl-1,2,3,4-tetrahydroisochinolin-3-carbonsäure nach Anspruch 1.

11. Verbindung 2-(3-Mercapto-2-mercaptomethylpropionyl)-6,7-methylendioxy-1,2,3,4-tetrahydroisochinolin-3-carbonsäure nach Anspruch 1.

12. Pharmazeutische Zusammensetzung, enthaltend als Wirkstoff eine wirksame Menge einer Verbindung nach Anspruch 1 bis 11 oder eines pharmazeutisch unbedenklichen Salzes derselben und einen pharmazeutisch unbedenklichen Träger, Exzipienten oder Verdünnungsstoff für diesen.

13. Verbindung der Formel (I) nach Anspruch 1 bis 11 oder ein pharmazeutisch unbedenkliches Salz derselben oder eine pharmazeutische Zusammensetzung nach Anspruch 12 zur Verwendung bei der Verhütung oder Behandlung von Hypertonie bei Säugern.

14. Verfahren zur Herstellung einer Verbindung der Formel (I) nach Anspruch 1 oder eines pharmazeutisch unbedenklichen Salzes derselben, dadurch gekennzeichnet, daß eine Verbindung der Formel (II) 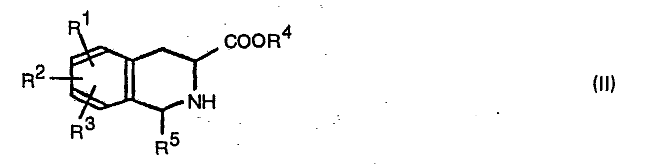 in der sämtliche Symbole die im Vorstehenden angegebene Bedetung haben, mit einer Verbindung der Formel (III) 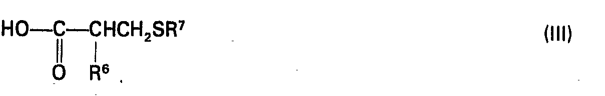 in der R⁶ und R⁷ die im vorstehenden angegebenen Bedeutungen haben, oder einem an der CarboxylFunktion reaktionsfähigen Derivat derselben zur Reaktion gebracht wird und gegebenenfalls die Verbindung der Formel (I) in das pharmazeutisch unbedenkliche Salze überführt wird.

## Patentansprüche (Patentansprüche für folgende(n) Vertragsstaat(en) : AT)

1. Verfahren zur Herstellung einer Verbindung der Formel (I) 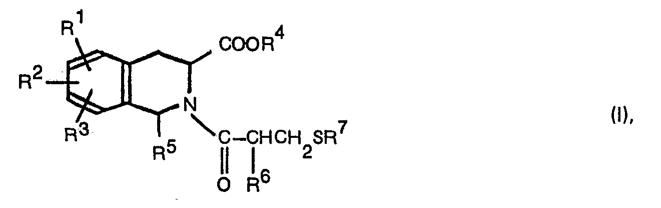 in der
R^{l}, R² und R³ gleich oder verschieden sein können und jeweils Wasserstoff, Halogen, Nitro, Hydroxyl, C₁- bis C₄-Alkyl, C₅- bis C₇-Cycloalkyl, C₁- bis C₄-Alkoxy oder 5- bis 7-gliedriges cyclisches Amino ausgewählt aus Pyrrolidinyl, Piperidino, Homopiperidinyl, Piperazinyl und Morpholino bezeichnen, mit der Maßgabe, daß, wenn R¹ und R² beide Wasserstoff sind, R³ einer der angegebenen, von Wasserstoff verschiedenen Substituenten ist, oder zwei beliebige, einander benachbarte Gruppen R¹, R² und R³ C₁- bis C₄-Alkylendioxy oder C₃- bis C₆-Alkylen bilden,
R⁴ und R⁵ jeweils Wasserstoff oder C₁- bis C₄₋Alkyl bezeichnen,
R⁶ Wasserstoff, C₁- bis C₄-Alkyl oder -CH₂SR⁸ ist, worin R⁸ Wasserstoff oder C₂- bis C₄₋Alkanoyl ist, und
R⁷ Wasserstoff, C₂- bis C₄-Alkanoyl oder Benzoyl ist, oder, wenn R⁶ ―CH₂SR⁸ ist, R⁷ und R⁸ gemeinsam eine Einfach-Bindung bezeichnen,
oder eines pharmazeutisch unbedenklichen Salzes derselben, dadurch gekennzeichnet, daß eine Verbindung der Formel (II) 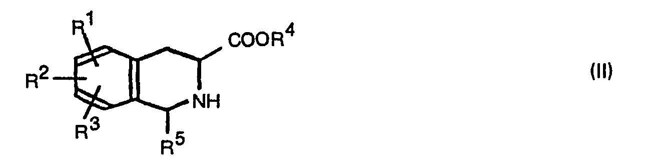 In der sämtliche Symbole die im Vorstehenden angegebene Bedeutung haben, mit einer Verbindung der Formel (III), 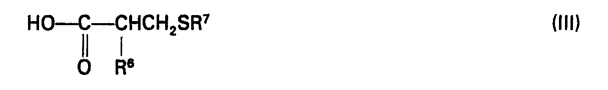 in der R^{6'}und R⁷ die im vorstehenden angegebenen Bedeutungen haben, oder einem an der CarboxylFunktion reaktionsfähigen Derivat derselben zur Reaktion gebracht wird und gegebenenfalls die Verbindung der Formel (I) in das pharmazeutisch unbedenkliche Salz Überführt wird.

2. Verfahren nach Anspruch 1, dadurch gekennzeichnet, daß R¹, R² und R³ gleich oder verschieden sein können und jeweils Wasserstoff, Hydroxyl, C₁- bis C₄-Alkyl oder C₁- bis C₄-Alkoxy bezeichnen, mit der Maßgabe, daß, wenn R' und R² beide Wasserstoff sind, R³ einer der angegebenen, von Wasserstoff verschiedenen Substituenten ist, oder zwei beliebige, einander benachbarte Gruppen R¹, R² und R³ C₁- bis C₄-Alkylendioxy bilden.

3. Verfahren nach Anspruch 2, dadurch gekennzeichnet, daß R' Wasserstoff ist und R² und R³ Hydroxyl oder C₁- bis C₄-Alkoxy in den 6- und 7-Stellungen sind oder R² und R³ zusammen Methylendioxy in den 6- und 7-Stellungen bezeichnen.

4. Verfahren nach Anspruch 2, dadurch gekennzeichnet, daß R' und R² Wasserstoff sind und R³ C,- bis C₄₋alkyl in der 7-Stellung ist.

5. Verfahren nach Anspruch 1 bis 4, dadurch gekennzeichnet, daß R⁴ Wasserstoff ist, R⁵ Wasserstoff oder Methyl ist, R⁶ Wasserstoff, Methyl oder ―CH₂SR⁸ ist, worin R⁸ Wasserstoff oder Acetyl ist, und R⁷ Wasserstoff oder Acetyl ist, oder, wenn R⁶ ―CH₂SR⁸ ist, R⁷ und R⁸ gemeinsam eine Einfach-Bindung bezeichnen.

6. Verfahren nach Anspruch 1, dadurch gekennzeichnet, daß die Verbindung der Formel (I) 2-(3-Acetylthio-2-methylpropionyl)-7-tert-butyl-1,2,3,4-tetrahydroisochinolin-3-carbonsäure ist.

7. Verfahren nach Anspruch 1, dadurch gekennzeichnet, daß die Verbindung der Formel (I) 7-tert-Butyl-2-(3-mercapto-2-methylpropionyl)-1,2,3,4-tetrahydroisochinolin-3-carbonsäure ist.

8. Verfahren nach Anspruch 1, dadurch gekennzeichnet, daß die Verbindung der Formel (I) 2-(3-Acetylthio-2-acetylthiomethylpropionyl)-7-tert-butyl-1,2,3,4-tetrahydroisochinolin-3-carbonsäure ist.

9. Verfahren nach Anspruch 1, dadurch gekennzeichnet, daß die Verbindung der Formel (II) 2-(3-Acetylthio-2-methylpropionyl)-7-isopropyl-1,2,3,4-tetrahydroisochinolin-3-carbonsäure ist.

10. Verfahren nach Anspruch 1, dadurch gekennzeichnet, daß die Verbindung der Formel (I) 2-(3-Acetylthio-2-acetylthiomethylpropionyl)-6,7-dihydroxy-1-methyl-1,2,3,4-tetrahydroisochinolin-3-carbonsäure ist.

11. Verfahren nach Anspruch 1, dadurch gekennzeichnet, daß die Verbindung der Formel (I) 2-(3-Mercapto-2-mercaptomethylpropionyl)-6,7-methylendioxy-1,2,3,4-tetrahydroisochinolin-3-carbonsäure ist.

12. Verbindung der Formel (I) nach Anspruch 1 bis 11 oder ein pharmazeutisch unbedenkliches Salz derselben oder eine pharmazeutische Zusammensetzung, die als Wirkstoff eine wirksame Menge einer Verbindung der Formel (I) nach Anspruch 1 bis 11 oder eines pharmazeutisch unbedenklichen Salzes derselben zusammen mit einem pharmazeutisch unbedenklichen Träger, Exzipienten oder Verdünnungsstoff für diesen enthält, zur Verwendung bei der Verhütung oder Behandlung von Hypertonie bei Säugem.
